# EUROPEAN PATENT APPLICATION

(11) **EP 3 960 756 A1**
(43) Date of publication of application: **02.03.2022**
(21) Application number: 20794486.9
(22) Date of filing: 22.04.2020
(51) Int. Cl.: C07K 14/195, A61K 39/39, A61K 47/68, A61P 1/00, A61P 7/00, A61P 17/14, A61P 19/08, A61P 19/10, A61P 27/12, A61P 43/00

(54) **FLAGELLIN FUSION PROTEIN AND USE THEREOF**

(30) Priority: 22.04.2019 KR 20190046866
(71) Applicant: Industry Foundation of Chonnam National University, Gwangju 61186 (KR)
(72) Inventor: CHO, Kyung A, Gwangju 61186 (KR); LIM, Jae Sung, Gwangju 61186 (KR)
(74) Representative: Hoefer & Partner Patentanwälte mbB
(86) International application number: PCT/KR2020/005327
(87) International publication number: WO 2020/218829

(57) **Abstract**

The present invention relates to a flagellin fusion proteins and a use thereof and, more specifically, to a fusion protein comprising flagellin, a fragment thereof; or a variant thereof; and an immunoglobulin Fc region and use in which a toll-like receptor 5 (TLR5) stimulating activity thereof is used. The fusion protein provided by the present invention has remarkably excellent toll-like receptor 5 (TLR5) pathway activation ability compared to wild-type flagellin, a fragment thereof, or a variant thereof, and therefore can be greatly utilized to develop a therapeutic agent and/or a vaccine adjuvant for a disease that can be prevented, improved, or treated through activation of the TLR5 pathway.

<110>    INDUSTRY FOUNDATION OF CHONNAM NATIONAL UNIVERSITY

<120>    Flagellin fusion protein and uses thereof

<130>    OP20-0027/PCT

<150>    KR 10-2019-0046866
<151>    2019-04-22

<160>    36

<170>    KoPatentIn 3.0

<210>    1
<211>    278
<212>    PRT
<213>    Artificial Sequence

<220>
<223>    Bacillus subtilis(Bs) flagellin


<400>    1
Met Arg Ile Asn His Asn Ile Ala Ala Leu Asn Thr Leu Asn Arg Leu
  1               5                  10                  15

Gly Ser Asn Asn Gly Ala Ala Gln Lys Asn Met Glu Lys Leu Ser Ser
             20                  25                  30

Gly Leu Arg Ile Asn Arg Ala Gly Asp Asp Ala Ala Gly Leu Ala Ile
         35                  40                  45

Ser Glu Lys Met Arg Gly Gln Ile Arg Gly Leu Glu Met Ala Ser Lys
     50                  55                  60

Asn Ser Gln Asp Gly Ile Ser Leu Ile Gln Thr Ala Glu Gly Ala Leu
 65                  70                  75                  80

Thr Glu Thr His Ala Ile Leu Gln Arg Met Arg Glu Leu Thr Val Gln
                 85                  90                  95

Ala Gly Asn Thr Gly Thr Gln Gln Ala Glu Asp Leu Gly Ala Ile Lys
            100                 105                 110

Asp Glu Met Asp Ala Leu Ile Glu Glu Ile Asp Gly Ile Ser Asn Arg
        115                 120                 125

Thr Glu Phe Asn Gly Lys Lys Leu Leu Asp Gly Thr Asn Ser Thr Asp
    130                 135                 140

Gly Phe Thr Phe Gln Ile Gly Ala Asn Ala Gly Gln Gln Leu Asn Val
145                 150                 155                 160

Lys Ile Asp Ser Met Ser Ser Thr Ala Leu Gly Val Asn Ala Leu Asp
                165                 170                 175

Val Thr Asp Phe Ala Ala Thr Ala Phe Asp Asp Gln Leu Lys Ser Ile
            180                 185                 190

Asp Thr Ala Ile Asn Thr Val Ser Thr Gln Arg Ala Lys Leu Gly Ala
        195                 200                 205

Val Gln Asn Arg Leu Glu His Thr Ile Asn Asn Leu Gly Ala Ser Gly

210                 215                 220

Glu Asn Leu Thr Ala Ala Glu Ser Arg Ile Arg Asp Val Asp Met Ala
225                 230                 235                 240

Lys Glu Met Ser Glu Phe Thr Lys Asn Asn Ile Leu Ser Gln Ala Ser
                245                 250                 255

Gln Ala Met Leu Ala Gln Ala Asn Gln Gln Pro Gln Asn Val Leu Gln
            260                 265                 270

Leu Leu Arg Pro Gly Lys
        275


<210>   2
<211>   505
<212>   PRT
<213>   Artificial Sequence

<220>
<223>   Salmonella dublin(Sd) flagellin


<400>   2
Met Ala Gln Val Ile Asn Thr Asn Ser Leu Ser Leu Leu Thr Gln Asn
 1               5                  10                  15

Asn Leu Asn Lys Ser Gln Ser Ser Leu Ser Ser Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ser Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ser
                85                  90                  95

Val Gln Ala Thr Asn Gly Thr Asn Ser Asp Ser Asp Leu Lys Ser Ile
            100                 105                 110

Gln Asp Glu Ile Gln Gln Arg Leu Glu Glu Ile Asp Arg Val Ser Asn
        115                 120                 125

Gln Thr Gln Phe Asn Gly Val Lys Val Leu Ser Gln Asp Asn Gln Met
    130                 135                 140

Lys Ile Gln Val Gly Ala Asn Asp Gly Glu Thr Ile Thr Ile Asp Leu
145                 150                 155                 160

Gln Lys Ile Asp Val Lys Ser Leu Gly Leu Asp Gly Phe Asn Val Asn
                165                 170                 175

Gly Pro Lys Glu Ala Thr Val Gly Asp Leu Lys Ser Ser Phe Lys Asn
            180                 185                 190

Val Thr Gly Tyr Asp Thr Tyr Ala Ala Gly Ala Asp Lys Tyr Arg Val
        195                 200                 205

Asp Ile Asn Ser Gly Ala Val Val Thr Asp Ala Val Ala Pro Asp Lys
    210                 215                 220

Val Tyr Val Asn Ala Ala Asn Gly Gln Leu Thr Thr Asp Asp Ala Glu
225                 230                 235                 240

Asn Asn Thr Ala Val Asp Leu Phe Lys Thr Thr Lys Ser Thr Ala Gly
                245                 250                 255

Thr Ala Glu Ala Lys Ala Ile Ala Gly Ala Ile Lys Gly Gly Lys Glu
            260                 265                 270

Gly Asp Thr Phe Asp Tyr Lys Gly Val Thr Phe Thr Ile Asp Thr Lys
        275                 280                 285

Thr Gly Asp Asp Gly Asn Gly Lys Val Ser Thr Thr Ile Asn Gly Glu
    290                 295                 300

Lys Val Thr Leu Thr Val Ala Asp Ile Ala Ile Gly Ala Ala Asp Val
305                 310                 315                 320

Asn Ala Ala Thr Leu Gln Ser Ser Lys Asn Val Tyr Thr Ser Val Val
                325                 330                 335

Asn Gly Gln Phe Thr Phe Asp Asp Lys Thr Lys Asn Glu Ser Ala Lys
            340                 345                 350

Leu Ser Asp Leu Glu Ala Asn Asn Ala Val Lys Gly Glu Ser Lys Ile
        355                 360                 365

Thr Val Asn Gly Ala Glu Tyr Thr Ala Asn Ala Thr Gly Asp Lys Ile
    370                 375                 380

Thr Leu Ala Gly Lys Thr Met Phe Ile Asp Lys Thr Ala Ser Gly Val
385                 390                 395                 400

Ser Thr Leu Ile Asn Glu Asp Ala Ala Ala Ala Lys Lys Ser Thr Ala
                405                 410                 415

Asn Pro Leu Ala Ser Ile Asp Ser Ala Leu Ser Lys Val Asp Ala Val
            420                 425                 430

Arg Ser Ser Leu Gly Ala Ile Gln Asn Arg Phe Asp Ser Ala Ile Thr
        435                 440                 445

Asn Leu Gly Asn Thr Val Thr Asn Leu Asn Ser Ala Arg Ser Arg Ile
    450                 455                 460

Glu Asp Ala Asp Tyr Ala Thr Glu Val Ser Asn Met Ser Lys Ala Gln
465                 470                 475                 480

Ile Leu Gln Gln Ala Gly Thr Ser Val Leu Ala Gln Ala Asn Gln Val
                485                 490                 495

Pro Gln Asn Val Leu Ser Leu Leu Arg
            500                 505


<210>    3
<211>    387
<212>    PRT
<213>    Artificial Sequence

<220>
<223>    Pseudomonas aeruginosa (Pa) flagellin


<400>    3
Met Ala Leu Thr Val Asn Thr Asn Ile Ala Ser Leu Asn Thr Gln Arg
1               5                   10                  15

Asn Gln Asn Asn Ser Ser Ala Ser Leu Asn Thr Ser Leu Gln Arg Leu
            20                  25                  30

Ser Thr Gly Ser Arg Ile Asn Ser Ala Lys Asp Asp Ala Ala Gly Leu
        35                  40                  45

Gln Ile Ala Asn Arg Leu Thr Ser Gln Val Asn Gly Leu Asn Val Ala
    50                  55                  60

Thr Lys Asn Ala Asn Asp Gly Ile Ser Leu Ala Gln Thr Ala Glu Gly
65                  70                  75                  80

Ala Leu Gln Gln Ser Thr Asn Ile Leu Gln Arg Met Arg Asp Leu Ser
                85                  90                  95

Leu Gln Ser Ala Asn Gly Ser Asn Ser Asp Ser Glu Arg Thr Ala Leu
            100                 105                 110

Asn Gly Glu Val Lys Gln Leu Gln Lys Glu Leu Asp Arg Ile Ser Asn
        115                 120                 125

Thr Thr Thr Phe Gly Gly Arg Lys Leu Leu Asp Gly Ser Phe Gly Val
    130                 135                 140

Ala Ser Phe Gln Val Gly Ser Ala Ala Asn Glu Ile Ile Ser Val Gly
145                 150                 155                 160

Ile Asp Glu Met Ser Ala Glu Ser Leu Asn Gly Thr Tyr Phe Lys Ala
                165                 170                 175

Asp Gly Gly Gly Ala Val Thr Ala Ala Thr Ala Ser Gly Thr Val Asp
            180                 185                 190

Ile Ala Ile Asp Ile Thr Gly Gly Ser Ala Val Asn Val Lys Val Asp
        195                 200                 205

Met Lys Gly Asn Glu Thr Ala Glu Gln Ala Ala Ala Lys Ile Ala Ala
    210                 215                 220

Ala Val Asn Asp Ala Asn Val Gly Ile Gly Ala Phe Thr Asp Gly Ala
225                 230                 235                 240

Gln Ile Ser Tyr Val Ser Lys Ala Ser Ala Asp Gly Thr Thr Ser Ala
                245                 250                 255

Val Ser Gly Val Ala Ile Thr Asp Thr Gly Ser Thr Gly Ala Gly Thr
            260                 265                 270

Ala Ala Gly Thr Thr Thr Phe Thr Glu Ala Asn Asp Thr Val Ala Lys
        275                 280                 285

Ile Asp Ile Ser Thr Ala Lys Gly Ala Gln Ser Ala Val Leu Val Ile
    290                 295                 300

Asp Glu Ala Ile Lys Gln Ile Asp Ala Gln Arg Ala Asp Leu Gly Ala
305                 310                 315                 320

Val Gln Asn Arg Phe Asp Asn Thr Ile Asn Asn Leu Lys Asn Ile Gly
                325                 330                 335

Glu Asn Val Ser Ala Ala Arg Gly Arg Ile Glu Asp Thr Asp Phe Ala
            340                 345                 350

Ala Glu Thr Ala Asn Leu Thr Lys Asn Gln Val Leu Gln Gln Ala Gly
        355                 360                 365

Thr Ala Ile Leu Ala Gln Ala Asn Gln Leu Pro Gln Ser Val Leu Ser
    370                 375                 380

Leu Leu Arg
385


<210>    4
<211>    550
<212>    PRT
<213>    Artificial Sequence

<220>
<223>    Shigella flexneri (Sf) flagellin


<400>    4
Met Ala Gln Val Ile Asn Thr Asn Ser Leu Ser Leu Ile Thr Gln Asn
  1               5                  10                  15

Asn Ile Asn Lys Asn Gln Ser Ala Leu Ser Ser Ser Ile Glu Arg Leu
             20                  25                  30

Ser Ser Gly Leu Arg Ile Asn Ser Ala Lys Asp Asp Ala Ala Gly Gln
         35                  40                  45

Ala Ile Ala Asn Arg Phe Thr Ser Asn Ile Lys Gly Leu Thr Gln Ala
     50                  55                  60

Ala Arg Asn Ala Asn Asp Gly Ile Ser Val Ala Gln Thr Thr Glu Gly
 65                  70                  75                  80

Ala Leu Ser Glu Ile Asn Asn Asn Leu Gln Arg Ile Arg Glu Leu Thr
                 85                  90                  95

Val Gln Ala Ser Thr Gly Thr Asn Ser Asp Ser Asp Leu Asp Ser Ile
            100                 105                 110

Gln Asp Glu Ile Lys Ser Arg Leu Asp Glu Ile Asp Arg Val Ser Gly
        115                 120                 125

Gln Thr Gln Phe Asn Gly Val Asn Val Leu Ala Lys Asp Gly Ser Met
    130                 135                 140

Lys Ile Gln Val Gly Ala Asn Asp Gly Gln Thr Ile Thr Ile Asp Leu
145                 150                 155                 160

Lys Lys Ile Asp Ser Asp Thr Leu Gly Leu Asn Gly Phe Asn Val Asn
                165                 170                 175

Gly Gly Gly Ala Val Ala Asn Thr Ala Ala Ser Lys Ala Asp Leu Val

180                 185                 190

Ala Ala Asn Ala Thr Val Val Gly Asn Lys Tyr Thr Val Ser Ala Gly
        195                 200                 205

Tyr Asp Ala Ala Lys Ala Ser Asp Leu Leu Ala Gly Val Ser Asp Gly
    210                 215                 220

Asp Thr Val Gln Ala Thr Ile Asn Asn Gly Phe Gly Thr Ala Ala Ser
225                 230                 235                 240

Ala Thr Asn Tyr Lys Tyr Asp Ser Ala Ser Lys Ser Tyr Ser Phe Asp
                245                 250                 255

Thr Thr Thr Ala Ser Ala Ala Asp Val Gln Lys Tyr Leu Thr Pro Gly
            260                 265                 270

Val Gly Asp Thr Ala Lys Gly Thr Ile Thr Ile Asp Gly Ser Ala Gln
        275                 280                 285

Asp Val Gln Ile Ser Ser Asp Gly Lys Ile Thr Ala Ser Asn Gly Asp
    290                 295                 300

Lys Leu Tyr Ile Asp Thr Thr Gly Arg Leu Thr Lys Asn Gly Ser Gly
305                 310                 315                 320

Ala Ser Leu Thr Glu Ala Ser Leu Ser Thr Leu Ala Ala Asn Asn Thr
                325                 330                 335

Lys Ala Thr Thr Ile Asp Ile Gly Gly Thr Ser Ile Ser Phe Thr Gly
            340                 345                 350

Asn Ser Thr Thr Pro Asp Thr Ile Thr Tyr Ser Val Thr Gly Ala Lys
        355                 360                 365

Val Asp Gln Ala Ala Phe Asp Lys Ala Val Ser Thr Ser Gly Asn Asn
    370                 375                 380

Val Asp Phe Thr Thr Ala Gly Tyr Ser Val Asn Gly Thr Thr Gly Ala
385                 390                 395                 400

Val Thr Lys Gly Val Asp Ser Val Tyr Val Asp Asn Asn Glu Ala Leu
                405                 410                 415

Thr Thr Ser Asp Thr Val Asp Phe Tyr Leu Gln Asp Asp Gly Ser Val
            420                 425                 430

Thr Asn Gly Ser Gly Lys Ala Val Tyr Lys Asp Ala Asp Gly Lys Leu
        435                 440                 445

Thr Thr Asp Ala Glu Thr Lys Ala Ala Thr Thr Ala Asp Pro Leu Lys
    450                 455                 460

Ala Leu Asp Glu Ala Ile Ser Ser Ile Asp Lys Phe Arg Ser Ser Leu
465                 470                 475                 480

Gly Ala Val Gln Asn Arg Leu Asp Ser Ala Val Thr Asn Leu Asn Asn
                485                 490                 495

Thr Thr Thr Asn Leu Ser Glu Ala Gln Ser Arg Ile Gln Asp Ala Asp
            500                 505                 510

Tyr Ala Thr Glu Val Ser Asn Met Ser Lys Ala Gln Ile Ile Gln Gln

515                 520                 525

Ala Gly Asn Ser Val Leu Ala Lys Ala Asn Gln Val Pro Gln Gln Val
    530                 535                 540

Leu Ser Leu Leu Gln Gly
545                 550


<210>    5
<211>    498
<212>    PRT
<213>    Artificial Sequence

<220>
<223>    Escherichia coli (Ec) flagellin


<400>    5
Met Ala Gln Val Ile Asn Thr Asn Ser Leu Ser Leu Ile Thr Gln Asn
  1               5                  10                  15

Asn Ile Asn Lys Asn Gln Ser Ala Leu Ser Ser Ser Ile Glu Arg Leu
             20                  25                  30

Ser Ser Gly Leu Arg Ile Asn Ser Ala Lys Asp Asp Ala Ala Gly Gln
         35                  40                  45

Ala Ile Ala Asn Arg Phe Thr Ser Asn Ile Lys Gly Leu Thr Gln Ala
    50                  55                  60

Ala Arg Asn Ala Asn Asp Gly Ile Ser Val Ala Gln Thr Thr Glu Gly
 65                  70                  75                  80

Ala Leu Ser Glu Ile Asn Asn Asn Leu Gln Arg Val Arg Glu Leu Thr
                 85                  90                  95

Val Gln Ala Thr Thr Gly Thr Asn Ser Glu Ser Asp Leu Ser Ser Ile
            100                 105                 110

Gln Asp Glu Ile Lys Ser Arg Leu Asp Glu Ile Asp Arg Val Ser Gly
        115                 120                 125

Gln Thr Gln Phe Asn Gly Val Asn Val Leu Ala Lys Asn Gly Ser Met
    130                 135                 140

Lys Ile Gln Val Gly Ala Asn Asp Asn Gln Thr Ile Thr Ile Asp Leu
145                 150                 155                 160

Lys Gln Ile Asp Ala Lys Thr Leu Gly Leu Asp Gly Phe Ser Val Lys
                165                 170                 175

Asn Asn Asp Thr Val Thr Thr Ser Ala Pro Val Thr Ala Phe Gly Ala
            180                 185                 190

Thr Thr Thr Asn Asn Ile Lys Leu Thr Gly Ile Thr Leu Ser Thr Glu
        195                 200                 205

Ala Ala Thr Asp Thr Gly Gly Thr Asn Pro Ala Ser Ile Glu Gly Val
    210                 215                 220

Tyr Thr Asp Asn Gly Asn Asp Tyr Tyr Ala Lys Ile Thr Gly Gly Asp
225                 230                 235                 240

Asn Asp Gly Lys Tyr Tyr Ala Val Thr Val Ala Asn Asp Gly Thr Val
                245                 250                 255

Thr Met Ala Thr Gly Ala Thr Ala Asn Ala Thr Val Thr Asp Ala Asn
            260                 265                 270

Thr Thr Lys Ala Thr Thr Ile Thr Ser Gly Gly Thr Pro Val Gln Ile
        275                 280                 285

Asp Asn Thr Ala Gly Ser Ala Thr Ala Asn Leu Gly Ala Val Ser Leu
    290                 295                 300

Val Lys Leu Gln Asp Ser Lys Gly Asn Asp Thr Asp Thr Tyr Ala Leu
305                 310                 315                 320

Lys Asp Thr Asn Gly Asn Leu Tyr Ala Ala Asp Val Asn Glu Thr Thr
                325                 330                 335

Gly Ala Val Ser Val Lys Thr Ile Thr Tyr Thr Asp Ser Ser Gly Ala
            340                 345                 350

Ala Ser Ser Pro Thr Ala Val Lys Leu Gly Gly Asp Asp Gly Lys Thr
        355                 360                 365

Glu Val Val Asp Ile Asp Gly Lys Thr Tyr Asp Ser Ala Asp Leu Asn
    370                 375                 380

Gly Gly Asn Leu Gln Thr Gly Leu Thr Ala Gly Gly Glu Ala Leu Thr
385                 390                 395                 400

Ala Val Ala Asn Gly Lys Thr Thr Asp Pro Leu Lys Ala Leu Asp Asp
                405                 410                 415

Ala Ile Ala Ser Val Asp Lys Phe Arg Ser Ser Leu Gly Ala Val Gln
            420                 425                 430

Asn Arg Leu Asp Ser Ala Val Thr Asn Leu Asn Asn Thr Thr Thr Asn
        435                 440                 445

Leu Ser Glu Ala Gln Ser Arg Ile Gln Asp Ala Asp Tyr Ala Thr Glu
    450                 455                 460

Val Ser Asn Met Ser Lys Ala Gln Ile Ile Gln Gln Ala Gly Asn Ser
465                 470                 475                 480

Val Leu Ala Lys Ala Asn Gln Val Pro Gln Gln Val Leu Ser Leu Leu
                485                 490                 495

Gln Gly

<210>    6
<211>    229
<212>    PRT
<213>    Artificial Sequence

<220>
<223>    Human IgG4 Fc region

<400>    6

Ala Met Val Arg Ser Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>   7
<211>   227
<212>   PRT
<213>   Artificial Sequence

<220>
<223>   Mouse IgG1 Fc region

<400>   7
Thr Met Val Arg Ser Gly Cys Lys Pro Cys Ile Cys Thr Val Pro Glu
1               5                   10                  15

Val Ser Ser Val Phe Ile Phe Pro Pro Lys Pro Lys Asp Val Leu Thr
            20                  25                  30

Ile Thr Leu Thr Pro Lys Val Thr Cys Val Val Val Asp Ile Ser Lys

35                  40                  45

Asp Asp Pro Glu Val Gln Phe Ser Trp Phe Val Asp Asp Val Glu Val
    50                  55                  60

His Thr Ala Gln Thr Gln Pro Arg Glu Glu Gln Phe Asn Ser Thr Phe
65                  70                  75                  80

Arg Ser Val Ser Glu Leu Pro Ile Met His Gln Asp Trp Leu Asn Gly
                85                  90                  95

Lys Glu Phe Lys Cys Arg Val Asn Ser Ala Ala Phe Pro Ala Pro Ile
            100                 105                 110

Glu Lys Thr Ile Ser Lys Thr Lys Gly Arg Pro Lys Ala Pro Gln Val
        115                 120                 125

Tyr Thr Ile Pro Pro Pro Lys Glu Gln Met Ala Lys Asp Lys Val Ser
    130                 135                 140

Leu Thr Cys Met Ile Thr Asp Phe Phe Pro Glu Asp Ile Thr Val Glu
145                 150                 155                 160

Trp Gln Trp Asn Gly Gln Pro Ala Glu Asn Tyr Lys Asn Thr Gln Pro
                165                 170                 175

Ile Met Asp Thr Asp Gly Ser Tyr Phe Val Tyr Ser Lys Leu Asn Val
            180                 185                 190

Gln Lys Ser Asn Trp Glu Ala Gly Asn Thr Phe Thr Cys Ser Val Leu
        195                 200                 205

His Glu Gly Leu His Asn His His Thr Glu Lys Ser Leu Ser His Ser
    210                 215                 220

Pro Gly Lys
225

<210>   8
<211>   5
<212>   PRT
<213>   Artificial Sequence

<220>
<223>   Linker 1

<400>   8
Gly Gly Gly Gly Ser
1               5

<210>   9
<211>   15
<212>   PRT
<213>   Artificial Sequence

<220>
<223>   Linker 2

<400>   9

Gly Gly Gly Gly Ser Gly Gly Gly Gly Ser Gly Gly Gly Gly Ser
1               5                   10                  15

<210>   10
<211>   509
<212>   PRT
<213>   Artificial Sequence

<220>
<223>   hIgG4-Fc-Bsflagellin fusion protein

<400>   10
Met Arg Ile Asn His Asn Ile Ala Ala Leu Asn Thr Leu Asn Arg Leu
1               5                   10                  15

Gly Ser Asn Asn Gly Ala Ala Gln Lys Asn Met Glu Lys Leu Ser Ser
            20                  25                  30

Gly Leu Arg Ile Asn Arg Ala Gly Asp Asp Ala Ala Gly Leu Ala Ile
        35                  40                  45

Ser Glu Lys Met Arg Gly Gln Ile Arg Gly Leu Glu Met Ala Ser Lys
    50                  55                  60

Asn Ser Gln Asp Gly Ile Ser Leu Ile Gln Thr Ala Glu Gly Ala Leu
65                  70                  75                  80

Thr Glu Thr His Ala Ile Leu Gln Arg Met Arg Glu Leu Thr Val Gln
                85                  90                  95

Ala Gly Asn Thr Gly Thr Gln Gln Ala Glu Asp Leu Gly Ala Ile Lys
            100                 105                 110

Asp Glu Met Asp Ala Leu Ile Glu Glu Ile Asp Gly Ile Ser Asn Arg
        115                 120                 125

Thr Glu Phe Asn Gly Lys Lys Leu Leu Asp Gly Thr Asn Ser Thr Asp
    130                 135                 140

Gly Phe Thr Phe Gln Ile Gly Ala Asn Ala Gly Gln Gln Leu Asn Val
145                 150                 155                 160

Lys Ile Asp Ser Met Ser Ser Thr Ala Leu Gly Val Asn Ala Leu Asp
                165                 170                 175

Val Thr Asp Phe Ala Ala Thr Ala Phe Asp Asp Gln Leu Lys Ser Ile
            180                 185                 190

Asp Thr Ala Ile Asn Thr Val Ser Thr Gln Arg Ala Lys Leu Gly Ala
        195                 200                 205

Val Gln Asn Arg Leu Glu His Thr Ile Asn Asn Leu Gly Ala Ser Gly
    210                 215                 220

Glu Asn Leu Thr Ala Ala Glu Ser Arg Ile Arg Asp Val Asp Met Ala
225                 230                 235                 240

Lys Glu Met Ser Glu Phe Thr Lys Asn Asn Ile Leu Ser Gln Ala Ser
                245                 250                 255

Gln Ala Met Leu Ala Gln Ala Asn Gln Gln Pro Gln Asn Val Leu Gln

260                 265                 270

Leu Leu Arg Gly Gly Gly Gly Ser Ala Met Val Arg Ser Pro Pro Cys
        275                 280                 285

Pro Ser Cys Pro Ala Pro Glu Phe Leu Gly Gly Pro Ser Val Phe Leu
    290                 295                 300

Phe Pro Pro Lys Pro Lys Asp Thr Leu Met Ile Ser Arg Thr Pro Glu
305                 310                 315                 320

Val Thr Cys Val Val Val Asp Val Ser Gln Glu Asp Pro Glu Val Gln
                325                 330                 335

Phe Asn Trp Tyr Val Asp Gly Val Glu Val His Asn Ala Lys Thr Lys
            340                 345                 350

Pro Arg Glu Glu Gln Phe Asn Ser Thr Tyr Arg Val Val Ser Val Leu
        355                 360                 365

Thr Val Leu His Gln Asp Trp Leu Asn Gly Lys Glu Tyr Lys Cys Lys
    370                 375                 380

Val Ser Asn Lys Gly Leu Pro Ser Ser Ile Glu Lys Thr Ile Ser Lys
385                 390                 395                 400

Ala Lys Gly Gln Pro Arg Glu Pro Gln Val Tyr Thr Leu Pro Pro Ser
                405                 410                 415

Gln Glu Glu Met Thr Lys Asn Gln Val Ser Leu Thr Cys Leu Val Lys
            420                 425                 430

Gly Phe Tyr Pro Ser Asp Ile Ala Val Glu Trp Glu Ser Asn Gly Gln
        435                 440                 445

Pro Glu Asn Asn Tyr Lys Thr Thr Pro Pro Val Leu Asp Ser Asp Gly
    450                 455                 460

Ser Phe Phe Leu Tyr Ser Arg Leu Thr Val Asp Lys Ser Arg Trp Gln
465                 470                 475                 480

Glu Gly Asn Val Phe Ser Cys Ser Val Met His Glu Ala Leu His Asn
                485                 490                 495

His Tyr Thr Gln Lys Ser Leu Ser Leu Ser Leu Gly Lys
            500                 505


<210>   11
<211>   507
<212>   PRT
<213>   Artificial Sequence

<220>
<223>   mIgG1-Fc-Bsflagellin fusion protein (with linker 1)


<400>   11
Met Arg Ile Asn His Asn Ile Ala Ala Leu Asn Thr Leu Asn Arg Leu
1               5                   10                  15

Gly Ser Asn Asn Gly Ala Ala Gln Lys Asn Met Glu Lys Leu Ser Ser
            20                  25                  30

Gly Leu Arg Ile Asn Arg Ala Gly Asp Asp Ala Ala Gly Leu Ala Ile
        35                  40                  45

Ser Glu Lys Met Arg Gly Gln Ile Arg Gly Leu Glu Met Ala Ser Lys
    50                  55                  60

Asn Ser Gln Asp Gly Ile Ser Leu Ile Gln Thr Ala Glu Gly Ala Leu
65                  70                  75                  80

Thr Glu Thr His Ala Ile Leu Gln Arg Met Arg Glu Leu Thr Val Gln
                85                  90                  95

Ala Gly Asn Thr Gly Thr Gln Gln Ala Glu Asp Leu Gly Ala Ile Lys
            100                 105                 110

Asp Glu Met Asp Ala Leu Ile Glu Glu Ile Asp Gly Ile Ser Asn Arg
        115                 120                 125

Thr Glu Phe Asn Gly Lys Lys Leu Leu Asp Gly Thr Asn Ser Thr Asp
    130                 135                 140

Gly Phe Thr Phe Gln Ile Gly Ala Asn Ala Gly Gln Gln Leu Asn Val
145                 150                 155                 160

Lys Ile Asp Ser Met Ser Ser Thr Ala Leu Gly Val Asn Ala Leu Asp
                165                 170                 175

Val Thr Asp Phe Ala Ala Thr Ala Phe Asp Asp Gln Leu Lys Ser Ile
            180                 185                 190

Asp Thr Ala Ile Asn Thr Val Ser Thr Gln Arg Ala Lys Leu Gly Ala
        195                 200                 205

Val Gln Asn Arg Leu Glu His Thr Ile Asn Asn Leu Gly Ala Ser Gly
    210                 215                 220

Glu Asn Leu Thr Ala Ala Glu Ser Arg Ile Arg Asp Val Asp Met Ala
225                 230                 235                 240

Lys Glu Met Ser Glu Phe Thr Lys Asn Asn Ile Leu Ser Gln Ala Ser
                245                 250                 255

Gln Ala Met Leu Ala Gln Ala Asn Gln Gln Pro Gln Asn Val Leu Gln
            260                 265                 270

Leu Leu Arg Gly Gly Gly Gly Ser Thr Met Val Arg Ser Gly Cys Lys
        275                 280                 285

Pro Cys Ile Cys Thr Val Pro Glu Val Ser Ser Val Phe Ile Phe Pro
    290                 295                 300

Pro Lys Pro Lys Asp Val Leu Thr Ile Thr Leu Thr Pro Lys Val Thr
305                 310                 315                 320

Cys Val Val Val Asp Ile Ser Lys Asp Asp Pro Glu Val Gln Phe Ser
                325                 330                 335

Trp Phe Val Asp Asp Val Glu Val His Thr Ala Gln Thr Gln Pro Arg
            340                 345                 350

Glu Glu Gln Phe Asn Ser Thr Phe Arg Ser Val Ser Glu Leu Pro Ile
        355                 360                 365

Met His Gln Asp Trp Leu Asn Gly Lys Glu Phe Lys Cys Arg Val Asn
    370                 375                 380

Ser Ala Ala Phe Pro Ala Pro Ile Glu Lys Thr Ile Ser Lys Thr Lys
385                 390                 395                 400

Gly Arg Pro Lys Ala Pro Gln Val Tyr Thr Ile Pro Pro Pro Lys Glu
                405                 410                 415

Gln Met Ala Lys Asp Lys Val Ser Leu Thr Cys Met Ile Thr Asp Phe
            420                 425                 430

Phe Pro Glu Asp Ile Thr Val Glu Trp Gln Trp Asn Gly Gln Pro Ala
        435                 440                 445

Glu Asn Tyr Lys Asn Thr Gln Pro Ile Met Asp Thr Asp Gly Ser Tyr
    450                 455                 460

Phe Val Tyr Ser Lys Leu Asn Val Gln Lys Ser Asn Trp Glu Ala Gly
465                 470                 475                 480

Asn Thr Phe Thr Cys Ser Val Leu His Glu Gly Leu His Asn His His
                485                 490                 495

Thr Glu Lys Ser Leu Ser His Ser Pro Gly Lys
            500                 505


<210>    12
<211>    517
<212>    PRT
<213>    Artificial Sequence

<220>
<223>    mIgG1-Fc-Bsflagellin fusion protein (with linker 2)


<400>    12
Met Arg Ile Asn His Asn Ile Ala Ala Leu Asn Thr Leu Asn Arg Leu
1               5                   10                  15

Gly Ser Asn Asn Gly Ala Ala Gln Lys Asn Met Glu Lys Leu Ser Ser
            20                  25                  30

Gly Leu Arg Ile Asn Arg Ala Gly Asp Asp Ala Ala Gly Leu Ala Ile
        35                  40                  45

Ser Glu Lys Met Arg Gly Gln Ile Arg Gly Leu Glu Met Ala Ser Lys
    50                  55                  60

Asn Ser Gln Asp Gly Ile Ser Leu Ile Gln Thr Ala Glu Gly Ala Leu
65                  70                  75                  80

Thr Glu Thr His Ala Ile Leu Gln Arg Met Arg Glu Leu Thr Val Gln
                85                  90                  95

Ala Gly Asn Thr Gly Thr Gln Gln Ala Glu Asp Leu Gly Ala Ile Lys
            100                 105                 110

Asp Glu Met Asp Ala Leu Ile Glu Glu Ile Asp Gly Ile Ser Asn Arg
        115                 120                 125

Thr Glu Phe Asn Gly Lys Lys Leu Leu Asp Gly Thr Asn Ser Thr Asp
    130                 135                 140

Gly Phe Thr Phe Gln Ile Gly Ala Asn Ala Gly Gln Gln Leu Asn Val
145                 150                 155                 160

Lys Ile Asp Ser Met Ser Ser Thr Ala Leu Gly Val Asn Ala Leu Asp
                165                 170                 175

Val Thr Asp Phe Ala Ala Thr Ala Phe Asp Asp Gln Leu Lys Ser Ile
            180                 185                 190

Asp Thr Ala Ile Asn Thr Val Ser Thr Gln Arg Ala Lys Leu Gly Ala
        195                 200                 205

Val Gln Asn Arg Leu Glu His Thr Ile Asn Asn Leu Gly Ala Ser Gly
    210                 215                 220

Glu Asn Leu Thr Ala Ala Glu Ser Arg Ile Arg Asp Val Asp Met Ala
225                 230                 235                 240

Lys Glu Met Ser Glu Phe Thr Lys Asn Asn Ile Leu Ser Gln Ala Ser
                245                 250                 255

Gln Ala Met Leu Ala Gln Ala Asn Gln Gln Pro Gln Asn Val Leu Gln
            260                 265                 270

Leu Leu Arg Gly Gly Gly Gly Ser Gly Gly Gly Gly Ser Gly Gly Gly
        275                 280                 285

Gly Ser Thr Met Val Arg Ser Gly Cys Lys Pro Cys Ile Cys Thr Val
    290                 295                 300

Pro Glu Val Ser Ser Val Phe Ile Phe Pro Pro Lys Pro Lys Asp Val
305                 310                 315                 320

Leu Thr Ile Thr Leu Thr Pro Lys Val Thr Cys Val Val Val Asp Ile
                325                 330                 335

Ser Lys Asp Asp Pro Glu Val Gln Phe Ser Trp Phe Val Asp Asp Val
            340                 345                 350

Glu Val His Thr Ala Gln Thr Gln Pro Arg Glu Glu Gln Phe Asn Ser
        355                 360                 365

Thr Phe Arg Ser Val Ser Glu Leu Pro Ile Met His Gln Asp Trp Leu
    370                 375                 380

Asn Gly Lys Glu Phe Lys Cys Arg Val Asn Ser Ala Ala Phe Pro Ala
385                 390                 395                 400

Pro Ile Glu Lys Thr Ile Ser Lys Thr Lys Gly Arg Pro Lys Ala Pro
                405                 410                 415

Gln Val Tyr Thr Ile Pro Pro Pro Lys Glu Gln Met Ala Lys Asp Lys
            420                 425                 430

Val Ser Leu Thr Cys Met Ile Thr Asp Phe Phe Pro Glu Asp Ile Thr
        435                 440                 445

Val Glu Trp Gln Trp Asn Gly Gln Pro Ala Glu Asn Tyr Lys Asn Thr
    450                 455                 460

Gln Pro Ile Met Asp Thr Asp Gly Ser Tyr Phe Val Tyr Ser Lys Leu
465                 470                 475                 480

Asn Val Gln Lys Ser Asn Trp Glu Ala Gly Asn Thr Phe Thr Cys Ser
                485                 490                 495

Val Leu His Glu Gly Leu His Asn His His Thr Glu Lys Ser Leu Ser
            500                 505                 510

His Ser Pro Gly Lys
        515


<210>   13
<211>   761
<212>   PRT
<213>   Artificial Sequence

<220>
<223>   hIgG4-Fc-Sdflagellin  fusion protein (with linker 1)


<400>   13
Met Tyr Arg Met Gln Leu Leu Ser Cys Ile Ala Leu Ser Leu Ala Leu
1               5                   10                  15

Val Thr Asn Ser Ile Ser Met Ala Gln Val Ile Asn Thr Asn Ser Leu
            20                  25                  30

Ser Leu Leu Thr Gln Asn Asn Leu Asn Lys Ser Gln Ser Ser Leu Ser
        35                  40                  45

Ser Ala Ile Glu Arg Leu Ser Ser Gly Leu Arg Ile Asn Ser Ala Lys
    50                  55                  60

Asp Asp Ala Ala Gly Gln Ala Ile Ala Asn Arg Phe Thr Ser Asn Ile
65                  70                  75                  80

Lys Gly Leu Thr Gln Ala Ser Arg Asn Ala Asn Asp Gly Ile Ser Ile
                85                  90                  95

Ala Gln Thr Thr Glu Gly Ala Leu Asn Glu Ile Asn Asn Asn Leu Gln
            100                 105                 110

Arg Val Arg Glu Leu Ser Val Gln Ala Thr Asn Gly Thr Asn Ser Asp
        115                 120                 125

Ser Asp Leu Lys Ser Ile Gln Asp Glu Ile Gln Gln Arg Leu Glu Glu
    130                 135                 140

Ile Asp Arg Val Ser Asn Gln Thr Gln Phe Asn Gly Val Lys Val Leu
145                 150                 155                 160

Ser Gln Asp Asn Gln Met Lys Ile Gln Val Gly Ala Asn Asp Gly Glu
                165                 170                 175

Thr Ile Thr Ile Asp Leu Gln Lys Ile Asp Val Lys Ser Leu Gly Leu
            180                 185                 190

Asp Gly Phe Asn Val Asn Gly Pro Lys Glu Ala Thr Val Gly Asp Leu
        195                 200                 205

Lys Ser Ser Phe Lys Asn Val Thr Gly Tyr Asp Thr Tyr Ala Ala Gly

210                 215                 220

Ala Asp Lys Tyr Arg Val Asp Ile Asn Ser Gly Ala Val Val Thr Asp
225                 230                 235                 240

Ala Val Ala Pro Asp Lys Val Tyr Val Asn Ala Ala Asn Gly Gln Leu
                245                 250                 255

Thr Thr Asp Asp Ala Glu Asn Asn Thr Ala Val Asp Leu Phe Lys Thr
            260                 265                 270

Thr Lys Ser Thr Ala Gly Thr Ala Glu Ala Lys Ala Ile Ala Gly Ala
        275                 280                 285

Ile Lys Gly Gly Lys Glu Gly Asp Thr Phe Asp Tyr Lys Gly Val Thr
    290                 295                 300

Phe Thr Ile Asp Thr Lys Thr Gly Asp Asp Gly Asn Gly Lys Val Ser
305                 310                 315                 320

Thr Thr Ile Asn Gly Glu Lys Val Thr Leu Thr Val Ala Asp Ile Ala
                325                 330                 335

Ile Gly Ala Ala Asp Val Asn Ala Ala Thr Leu Gln Ser Ser Lys Asn
            340                 345                 350

Val Tyr Thr Ser Val Val Asn Gly Gln Phe Thr Phe Asp Asp Lys Thr
        355                 360                 365

Lys Asn Glu Ser Ala Lys Leu Ser Asp Leu Glu Ala Asn Asn Ala Val
    370                 375                 380

Lys Gly Glu Ser Lys Ile Thr Val Asn Gly Ala Glu Tyr Thr Ala Asn
385                 390                 395                 400

Ala Thr Gly Asp Lys Ile Thr Leu Ala Gly Lys Thr Met Phe Ile Asp
                405                 410                 415

Lys Thr Ala Ser Gly Val Ser Thr Leu Ile Asn Glu Asp Ala Ala Ala
            420                 425                 430

Ala Lys Lys Ser Thr Ala Asn Pro Leu Ala Ser Ile Asp Ser Ala Leu
        435                 440                 445

Ser Lys Val Asp Ala Val Arg Ser Ser Leu Gly Ala Ile Gln Asn Arg
    450                 455                 460

Phe Asp Ser Ala Ile Thr Asn Leu Gly Asn Thr Val Thr Asn Leu Asn
465                 470                 475                 480

Ser Ala Arg Ser Arg Ile Glu Asp Ala Asp Tyr Ala Thr Glu Val Ser
                485                 490                 495

Asn Met Ser Lys Ala Gln Ile Leu Gln Gln Ala Gly Thr Ser Val Leu
            500                 505                 510

Ala Gln Ala Asn Gln Val Pro Gln Asn Val Leu Ser Leu Leu Arg Gly
        515                 520                 525

Gly Gly Gly Ser Ala Met Val Arg Ser Pro Pro Cys Pro Ser Cys Pro
    530                 535                 540

Ala Pro Glu Phe Leu Gly Gly Pro Ser Val Phe Leu Phe Pro Pro Lys

545                 550                 555                 560

Pro Lys Asp Thr Leu Met Ile Ser Arg Thr Pro Glu Val Thr Cys Val
                565                 570                 575

Val Val Asp Val Ser Gln Glu Asp Pro Glu Val Gln Phe Asn Trp Tyr
            580                 585                 590

Val Asp Gly Val Glu Val His Asn Ala Lys Thr Lys Pro Arg Glu Glu
        595                 600                 605

Gln Phe Asn Ser Thr Tyr Arg Val Val Ser Val Leu Thr Val Leu His
    610                 615                 620

Gln Asp Trp Leu Asn Gly Lys Glu Tyr Lys Cys Lys Val Ser Asn Lys
625                 630                 635                 640

Gly Leu Pro Ser Ser Ile Glu Lys Thr Ile Ser Lys Ala Lys Gly Gln
                645                 650                 655

Pro Arg Glu Pro Gln Val Tyr Thr Leu Pro Pro Ser Gln Glu Glu Met
            660                 665                 670

Thr Lys Asn Gln Val Ser Leu Thr Cys Leu Val Lys Gly Phe Tyr Pro
        675                 680                 685

Ser Asp Ile Ala Val Glu Trp Glu Ser Asn Gly Gln Pro Glu Asn Asn
    690                 695                 700

Tyr Lys Thr Thr Pro Pro Val Leu Asp Ser Asp Gly Ser Phe Phe Leu
705                 710                 715                 720

Tyr Ser Arg Leu Thr Val Asp Lys Ser Arg Trp Gln Glu Gly Asn Val
                725                 730                 735

Phe Ser Cys Ser Val Met His Glu Ala Leu His Asn His Tyr Thr Gln
            740                 745                 750

Lys Ser Leu Ser Leu Ser Leu Gly Lys
        755                 760


<210>   14
<211>   643
<212>   PRT
<213>   Artificial Sequence

<220>
<223>   hIgG4-Fc-Paflagellin  fusion protein (with linker 1)


<400>   14
Met Tyr Arg Met Gln Leu Leu Ser Cys Ile Ala Leu Ser Leu Ala Leu
1               5                   10                  15

Val Thr Asn Ser Ile Ser Met Ala Leu Thr Val Asn Thr Asn Ile Ala
            20                  25                  30

Ser Leu Asn Thr Gln Arg Asn Gln Asn Asn Ser Ser Ala Ser Leu Asn
        35                  40                  45

Thr Ser Leu Gln Arg Leu Ser Thr Gly Ser Arg Ile Asn Ser Ala Lys
    50                  55                  60

Asp Asp Ala Ala Gly Leu Gln Ile Ala Asn Arg Leu Thr Ser Gln Val
65                  70                  75                  80

Asn Gly Leu Asn Val Ala Thr Lys Asn Ala Asn Asp Gly Ile Ser Leu
                85                  90                  95

Ala Gln Thr Ala Glu Gly Ala Leu Gln Gln Ser Thr Asn Ile Leu Gln
            100                 105                 110

Arg Met Arg Asp Leu Ser Leu Gln Ser Ala Asn Gly Ser Asn Ser Asp
        115                 120                 125

Ser Glu Arg Thr Ala Leu Asn Gly Glu Val Lys Gln Leu Gln Lys Glu
    130                 135                 140

Leu Asp Arg Ile Ser Asn Thr Thr Thr Phe Gly Gly Arg Lys Leu Leu
145                 150                 155                 160

Asp Gly Ser Phe Gly Val Ala Ser Phe Gln Val Gly Ser Ala Ala Asn
                165                 170                 175

Glu Ile Ile Ser Val Gly Ile Asp Glu Met Ser Ala Glu Ser Leu Asn
            180                 185                 190

Gly Thr Tyr Phe Lys Ala Asp Gly Gly Gly Ala Val Thr Ala Ala Thr
        195                 200                 205

Ala Ser Gly Thr Val Asp Ile Ala Ile Asp Ile Thr Gly Gly Ser Ala
    210                 215                 220

Val Asn Val Lys Val Asp Met Lys Gly Asn Glu Thr Ala Glu Gln Ala
225                 230                 235                 240

Ala Ala Lys Ile Ala Ala Ala Val Asn Asp Ala Asn Val Gly Ile Gly
                245                 250                 255

Ala Phe Thr Asp Gly Ala Gln Ile Ser Tyr Val Ser Lys Ala Ser Ala
            260                 265                 270

Asp Gly Thr Thr Ser Ala Val Ser Gly Val Ala Ile Thr Asp Thr Gly
        275                 280                 285

Ser Thr Gly Ala Gly Thr Ala Ala Gly Thr Thr Thr Phe Thr Glu Ala
    290                 295                 300

Asn Asp Thr Val Ala Lys Ile Asp Ile Ser Thr Ala Lys Gly Ala Gln
305                 310                 315                 320

Ser Ala Val Leu Val Ile Asp Glu Ala Ile Lys Gln Ile Asp Ala Gln
                325                 330                 335

Arg Ala Asp Leu Gly Ala Val Gln Asn Arg Phe Asp Asn Thr Ile Asn
            340                 345                 350

Asn Leu Lys Asn Ile Gly Glu Asn Val Ser Ala Ala Arg Gly Arg Ile
        355                 360                 365

Glu Asp Thr Asp Phe Ala Ala Glu Thr Ala Asn Leu Thr Lys Asn Gln
    370                 375                 380

Val Leu Gln Gln Ala Gly Thr Ala Ile Leu Ala Gln Ala Asn Gln Leu
385                 390                 395                 400

Pro Gln Ser Val Leu Ser Leu Leu Arg Gly Gly Gly Gly Ser Ala Met
                405                 410                 415

Val Arg Ser Pro Pro Cys Pro Ser Cys Pro Ala Pro Glu Phe Leu Gly
            420                 425                 430

Gly Pro Ser Val Phe Leu Phe Pro Pro Lys Pro Lys Asp Thr Leu Met
        435                 440                 445

Ile Ser Arg Thr Pro Glu Val Thr Cys Val Val Val Asp Val Ser Gln
    450                 455                 460

Glu Asp Pro Glu Val Gln Phe Asn Trp Tyr Val Asp Gly Val Glu Val
465                 470                 475                 480

His Asn Ala Lys Thr Lys Pro Arg Glu Glu Gln Phe Asn Ser Thr Tyr
                485                 490                 495

Arg Val Val Ser Val Leu Thr Val Leu His Gln Asp Trp Leu Asn Gly
            500                 505                 510

Lys Glu Tyr Lys Cys Lys Val Ser Asn Lys Gly Leu Pro Ser Ser Ile
        515                 520                 525

Glu Lys Thr Ile Ser Lys Ala Lys Gly Gln Pro Arg Glu Pro Gln Val
    530                 535                 540

Tyr Thr Leu Pro Pro Ser Gln Glu Glu Met Thr Lys Asn Gln Val Ser
545                 550                 555                 560

Leu Thr Cys Leu Val Lys Gly Phe Tyr Pro Ser Asp Ile Ala Val Glu
                565                 570                 575

Trp Glu Ser Asn Gly Gln Pro Glu Asn Asn Tyr Lys Thr Thr Pro Pro
            580                 585                 590

Val Leu Asp Ser Asp Gly Ser Phe Phe Leu Tyr Ser Arg Leu Thr Val
        595                 600                 605

Asp Lys Ser Arg Trp Gln Glu Gly Asn Val Phe Ser Cys Ser Val Met
    610                 615                 620

His Glu Ala Leu His Asn His Tyr Thr Gln Lys Ser Leu Ser Leu Ser
625                 630                 635                 640

Leu Gly Lys



<210>   15
<211>   806
<212>   PRT
<213>   Artificial Sequence

<220>
<223>   hIgG4-Fc-Sfflagellin fusion protein (with linker 1)


<400>   15
Met Tyr Arg Met Gln Leu Leu Ser Cys Ile Ala Leu Ser Leu Ala Leu
1               5                   10                  15

Val Thr Asn Ser Ile Ser Met Ala Gln Val Ile Asn Thr Asn Ser Leu
                20                  25                  30

Ser Leu Ile Thr Gln Asn Asn Ile Asn Lys Asn Gln Ser Ala Leu Ser
            35                  40                  45

Ser Ser Ile Glu Arg Leu Ser Ser Gly Leu Arg Ile Asn Ser Ala Lys
    50                  55                  60

Asp Asp Ala Ala Gly Gln Ala Ile Ala Asn Arg Phe Thr Ser Asn Ile
65                  70                  75                  80

Lys Gly Leu Thr Gln Ala Ala Arg Asn Ala Asn Asp Gly Ile Ser Val
                85                  90                  95

Ala Gln Thr Thr Glu Gly Ala Leu Ser Glu Ile Asn Asn Asn Leu Gln
            100                 105                 110

Arg Ile Arg Glu Leu Thr Val Gln Ala Ser Thr Gly Thr Asn Ser Asp
        115                 120                 125

Ser Asp Leu Asp Ser Ile Gln Asp Glu Ile Lys Ser Arg Leu Asp Glu
    130                 135                 140

Ile Asp Arg Val Ser Gly Gln Thr Gln Phe Asn Gly Val Asn Val Leu
145                 150                 155                 160

Ala Lys Asp Gly Ser Met Lys Ile Gln Val Gly Ala Asn Asp Gly Gln
                165                 170                 175

Thr Ile Thr Ile Asp Leu Lys Lys Ile Asp Ser Asp Thr Leu Gly Leu
            180                 185                 190

Asn Gly Phe Asn Val Asn Gly Gly Gly Ala Val Ala Asn Thr Ala Ala
        195                 200                 205

Ser Lys Ala Asp Leu Val Ala Ala Asn Ala Thr Val Val Gly Asn Lys
    210                 215                 220

Tyr Thr Val Ser Ala Gly Tyr Asp Ala Ala Lys Ala Ser Asp Leu Leu
225                 230                 235                 240

Ala Gly Val Ser Asp Gly Asp Thr Val Gln Ala Thr Ile Asn Asn Gly
                245                 250                 255

Phe Gly Thr Ala Ala Ser Ala Thr Asn Tyr Lys Tyr Asp Ser Ala Ser
            260                 265                 270

Lys Ser Tyr Ser Phe Asp Thr Thr Thr Ala Ser Ala Ala Asp Val Gln
        275                 280                 285

Lys Tyr Leu Thr Pro Gly Val Gly Asp Thr Ala Lys Gly Thr Ile Thr
    290                 295                 300

Ile Asp Gly Ser Ala Gln Asp Val Gln Ile Ser Ser Asp Gly Lys Ile
305                 310                 315                 320

Thr Ala Ser Asn Gly Asp Lys Leu Tyr Ile Asp Thr Thr Gly Arg Leu
                325                 330                 335

Thr Lys Asn Gly Ser Gly Ala Ser Leu Thr Glu Ala Ser Leu Ser Thr
            340                 345                 350

Leu Ala Ala Asn Asn Thr Lys Ala Thr Thr Ile Asp Ile Gly Gly Thr
        355                 360                 365

Ser Ile Ser Phe Thr Gly Asn Ser Thr Thr Pro Asp Thr Ile Thr Tyr
    370                 375                 380

Ser Val Thr Gly Ala Lys Val Asp Gln Ala Ala Phe Asp Lys Ala Val
385                 390                 395                 400

Ser Thr Ser Gly Asn Asn Val Asp Phe Thr Thr Ala Gly Tyr Ser Val
                405                 410                 415

Asn Gly Thr Thr Gly Ala Val Thr Lys Gly Val Asp Ser Val Tyr Val
            420                 425                 430

Asp Asn Asn Glu Ala Leu Thr Thr Ser Asp Thr Val Asp Phe Tyr Leu
        435                 440                 445

Gln Asp Asp Gly Ser Val Thr Asn Gly Ser Gly Lys Ala Val Tyr Lys
    450                 455                 460

Asp Ala Asp Gly Lys Leu Thr Thr Asp Ala Glu Thr Lys Ala Ala Thr
465                 470                 475                 480

Thr Ala Asp Pro Leu Lys Ala Leu Asp Glu Ala Ile Ser Ser Ile Asp
                485                 490                 495

Lys Phe Arg Ser Ser Leu Gly Ala Val Gln Asn Arg Leu Asp Ser Ala
            500                 505                 510

Val Thr Asn Leu Asn Asn Thr Thr Thr Asn Leu Ser Glu Ala Gln Ser
        515                 520                 525

Arg Ile Gln Asp Ala Asp Tyr Ala Thr Glu Val Ser Asn Met Ser Lys
    530                 535                 540

Ala Gln Ile Ile Gln Gln Ala Gly Asn Ser Val Leu Ala Lys Ala Asn
545                 550                 555                 560

Gln Val Pro Gln Gln Val Leu Ser Leu Leu Gln Gly Gly Gly Gly Gly
                565                 570                 575

Ser Ala Met Val Arg Ser Pro Pro Cys Pro Ser Cys Pro Ala Pro Glu
            580                 585                 590

Phe Leu Gly Gly Pro Ser Val Phe Leu Phe Pro Pro Lys Pro Lys Asp
        595                 600                 605

Thr Leu Met Ile Ser Arg Thr Pro Glu Val Thr Cys Val Val Val Asp
    610                 615                 620

Val Ser Gln Glu Asp Pro Glu Val Gln Phe Asn Trp Tyr Val Asp Gly
625                 630                 635                 640

Val Glu Val His Asn Ala Lys Thr Lys Pro Arg Glu Glu Gln Phe Asn
                645                 650                 655

Ser Thr Tyr Arg Val Val Ser Val Leu Thr Val Leu His Gln Asp Trp
            660                 665                 670

Leu Asn Gly Lys Glu Tyr Lys Cys Lys Val Ser Asn Lys Gly Leu Pro
        675                 680                 685

Ser Ser Ile Glu Lys Thr Ile Ser Lys Ala Lys Gly Gln Pro Arg Glu
    690                 695                 700

Pro Gln Val Tyr Thr Leu Pro Pro Ser Gln Glu Glu Met Thr Lys Asn
705                 710                 715                 720

Gln Val Ser Leu Thr Cys Leu Val Lys Gly Phe Tyr Pro Ser Asp Ile
                725                 730                 735

Ala Val Glu Trp Glu Ser Asn Gly Gln Pro Glu Asn Asn Tyr Lys Thr
            740                 745                 750

Thr Pro Pro Val Leu Asp Ser Asp Gly Ser Phe Phe Leu Tyr Ser Arg
        755                 760                 765

Leu Thr Val Asp Lys Ser Arg Trp Gln Glu Gly Asn Val Phe Ser Cys
    770                 775                 780

Ser Val Met His Glu Ala Leu His Asn His Tyr Thr Gln Lys Ser Leu
785                 790                 795                 800

Ser Leu Ser Leu Gly Lys
                805


<210>   16
<211>   754
<212>   PRT
<213>   Artificial Sequence

<220>
<223>   hIgG4-Fc-Ecflagellin fusion protein (with linker 1)


<400>   16
Met Tyr Arg Met Gln Leu Leu Ser Cys Ile Ala Leu Ser Leu Ala Leu
  1               5                  10                  15

Val Thr Asn Ser Ile Ser Met Ala Gln Val Ile Asn Thr Asn Ser Leu
             20                  25                  30

Ser Leu Ile Thr Gln Asn Asn Ile Asn Lys Asn Gln Ser Ala Leu Ser
         35                  40                  45

Ser Ser Ile Glu Arg Leu Ser Ser Gly Leu Arg Ile Asn Ser Ala Lys
     50                  55                  60

Asp Asp Ala Ala Gly Gln Ala Ile Ala Asn Arg Phe Thr Ser Asn Ile
 65                  70                  75                  80

Lys Gly Leu Thr Gln Ala Ala Arg Asn Ala Asn Asp Gly Ile Ser Val
                 85                  90                  95

Ala Gln Thr Thr Glu Gly Ala Leu Ser Glu Ile Asn Asn Asn Leu Gln
            100                 105                 110

Arg Val Arg Glu Leu Thr Val Gln Ala Thr Thr Gly Thr Asn Ser Glu
        115                 120                 125

Ser Asp Leu Ser Ser Ile Gln Asp Glu Ile Lys Ser Arg Leu Asp Glu
    130                 135                 140

Ile Asp Arg Val Ser Gly Gln Thr Gln Phe Asn Gly Val Asn Val Leu

145                 150                 155                 160

Ala Lys Asn Gly Ser Met Lys Ile Gln Val Gly Ala Asn Asp Asn Gln
                165                 170                 175

Thr Ile Thr Ile Asp Leu Lys Gln Ile Asp Ala Lys Thr Leu Gly Leu
            180                 185                 190

Asp Gly Phe Ser Val Lys Asn Asn Asp Thr Val Thr Thr Ser Ala Pro
        195                 200                 205

Val Thr Ala Phe Gly Ala Thr Thr Thr Asn Asn Ile Lys Leu Thr Gly
    210                 215                 220

Ile Thr Leu Ser Thr Glu Ala Ala Thr Asp Thr Gly Gly Thr Asn Pro
225                 230                 235                 240

Ala Ser Ile Glu Gly Val Tyr Thr Asp Asn Gly Asn Asp Tyr Tyr Ala
                245                 250                 255

Lys Ile Thr Gly Gly Asp Asn Asp Gly Lys Tyr Tyr Ala Val Thr Val
            260                 265                 270

Ala Asn Asp Gly Thr Val Thr Met Ala Thr Gly Ala Thr Ala Asn Ala
        275                 280                 285

Thr Val Thr Asp Ala Asn Thr Thr Lys Ala Thr Thr Ile Thr Ser Gly
    290                 295                 300

Gly Thr Pro Val Gln Ile Asp Asn Thr Ala Gly Ser Ala Thr Ala Asn
305                 310                 315                 320

Leu Gly Ala Val Ser Leu Val Lys Leu Gln Asp Ser Lys Gly Asn Asp
                325                 330                 335

Thr Asp Thr Tyr Ala Leu Lys Asp Thr Asn Gly Asn Leu Tyr Ala Ala
            340                 345                 350

Asp Val Asn Glu Thr Thr Gly Ala Val Ser Val Lys Thr Ile Thr Tyr
        355                 360                 365

Thr Asp Ser Ser Gly Ala Ala Ser Ser Pro Thr Ala Val Lys Leu Gly
    370                 375                 380

Gly Asp Asp Gly Lys Thr Glu Val Val Asp Ile Asp Gly Lys Thr Tyr
385                 390                 395                 400

Asp Ser Ala Asp Leu Asn Gly Gly Asn Leu Gln Thr Gly Leu Thr Ala
                405                 410                 415

Gly Gly Glu Ala Leu Thr Ala Val Ala Asn Gly Lys Thr Thr Asp Pro
            420                 425                 430

Leu Lys Ala Leu Asp Asp Ala Ile Ala Ser Val Asp Lys Phe Arg Ser
        435                 440                 445

Ser Leu Gly Ala Val Gln Asn Arg Leu Asp Ser Ala Val Thr Asn Leu
    450                 455                 460

Asn Asn Thr Thr Thr Asn Leu Ser Glu Ala Gln Ser Arg Ile Gln Asp
465                 470                 475                 480

Ala Asp Tyr Ala Thr Glu Val Ser Asn Met Ser Lys Ala Gln Ile Ile

485                 490                 495

Gln Gln Ala Gly Asn Ser Val Leu Ala Lys Ala Asn Gln Val Pro Gln
            500                 505                 510

Gln Val Leu Ser Leu Leu Gln Gly Gly Gly Gly Gly Ser Ala Met Val
        515                 520                 525

Arg Ser Pro Pro Cys Pro Ser Cys Pro Ala Pro Glu Phe Leu Gly Gly
    530                 535                 540

Pro Ser Val Phe Leu Phe Pro Pro Lys Pro Lys Asp Thr Leu Met Ile
545                 550                 555                 560

Ser Arg Thr Pro Glu Val Thr Cys Val Val Val Asp Val Ser Gln Glu
                565                 570                 575

Asp Pro Glu Val Gln Phe Asn Trp Tyr Val Asp Gly Val Glu Val His
            580                 585                 590

Asn Ala Lys Thr Lys Pro Arg Glu Glu Gln Phe Asn Ser Thr Tyr Arg
        595                 600                 605

Val Val Ser Val Leu Thr Val Leu His Gln Asp Trp Leu Asn Gly Lys
    610                 615                 620

Glu Tyr Lys Cys Lys Val Ser Asn Lys Gly Leu Pro Ser Ser Ile Glu
625                 630                 635                 640

Lys Thr Ile Ser Lys Ala Lys Gly Gln Pro Arg Glu Pro Gln Val Tyr
                645                 650                 655

Thr Leu Pro Pro Ser Gln Glu Glu Met Thr Lys Asn Gln Val Ser Leu
            660                 665                 670

Thr Cys Leu Val Lys Gly Phe Tyr Pro Ser Asp Ile Ala Val Glu Trp
        675                 680                 685

Glu Ser Asn Gly Gln Pro Glu Asn Asn Tyr Lys Thr Thr Pro Pro Val
    690                 695                 700

Leu Asp Ser Asp Gly Ser Phe Phe Leu Tyr Ser Arg Leu Thr Val Asp
705                 710                 715                 720

Lys Ser Arg Trp Gln Glu Gly Asn Val Phe Ser Cys Ser Val Met His
                725                 730                 735

Glu Ala Leu His Asn His Tyr Thr Gln Lys Ser Leu Ser Leu Ser Leu
            740                 745                 750

Gly Lys

<210>   17
<211>   1596
<212>   DNA
<213>   Artificial Sequence

<220>
<223>   polynucleotide encoding hIgG4-Fc-Bsflagellin

<400>    17
atgtacagga tgcaactcct gtcttgcatt gcactaagtc ttgcacttgt cacgaattcg      60

atatcgatga gaattaacca caatattgca gcacttaaca cattgaatcg tttgggttca     120

aacaacggag cagcacaaaa gaatatggag aagctttctt caggtcttcg tatcaaccgt     180

gcgggagatg acgcagcagg tctagcgatc tctgaaaaaa tgagaggaca aatcagaggt     240

cttgaaatgg cttctaaaaa ctctcaagat ggaatctctc ttatccaaac agctgagggt     300

gcattaactg aaactcatgc aattcttcaa cgtatgcgtg aacttactgt tcaagcagga     360

aacacaggta cacaacaagc tgaagatctt ggtgcaatta aagatgaaat ggatgcgctt     420

atcgaggaaa ttgatggcat ttcaaaccgt actgaattta acggtaaaaa gttgctagac     480

ggaactaatt ctactgatgg tttcacattc caaattggtg cgaatgctgg ccaacaacta     540

aatgtaaaaa ttgacagcat gtcatcaact gctttaggag taaacgcact tgatgtaaca     600

gatttcgctg ctactgcttt tgatgatcaa cttaaaagta ttgatactgc catcaatact     660

gtatcaactc aacgtgctaa attaggtgcg gtacaaaacc gtctagagca tacaatcaac     720

aacttaggcg cttctggtga aaacctgaca gctgctgagt ctcgtatccg tgacgttgac     780

atggctaaag aaatgagcga gttcactaag aacaacattc tttctcaagc ttctcaagct     840

atgcttgctc aagctaacca acagcctcaa aacgtacttc aattattacg tggtggcgga     900

gggtctgcca tggttagatc tcccccatgc ccatcatgcc cagcacctga gttcctgggg     960

ggaccatcag tcttcctgtt ccccccaaaa cccaaggaca ctctcatgat ctcccggacc    1020

cctgaggtca cgtgcgtggt ggtggacgtg agccaggaag accccgaggt ccagttcaac    1080

tggtacgtgg atggcgtgga ggtgcataat gccaagacaa agccgcggga ggagcagttc    1140

aacagcacgt accgtgtggt cagcgtcctc accgtcctgc accaggactg gctgaacggc    1200

aaggagtaca agtgcaaggt ctccaacaaa ggcctcccgt cctccatcga gaaaaccatc    1260

tccaaagcca aagggcagcc ccgagagcca caggtgtaca ccctgccccc atcccaggag    1320

gagatgacca agaaccaggt cagcctgacc tgcctggtca aaggcttcta ccccagcgac    1380

atcgccgtgg agtgggagag caatgggcag ccggagaaca actacaagac cacgcctccc    1440

gtgctggact ccgacggctc cttcttcctc tacagcaggc taaccgtgga caagagcagg    1500

tggcaggagg ggaatgtctt ctcatgctcc gtgatgcatg aggctctgca caaccactac    1560

acacagaaga gcctctccct gtctctgggt aaataa                              1596

<210>    18
<211>    1524
<212>    DNA
<213>    Artificial Sequence

<220>
<223>    polynucleotide encoding mIgG1-Fc-Bsflagellin (with linker 1)

<400>    18
atgagaatta accacaatat tgcagcactt aacacattga atcgtttggg ttcaaacaac      60

ggagcagcac aaaagaatat ggagaagctt tcttcaggtc ttcgtatcaa ccgtgcggga     120

gatgacgcag caggtctagc gatctctgaa aaaatgagag gacaaatcag aggtcttgaa     180

atggcttcta aaaactctca agatggaatc tctcttatcc aaacagctga gggtgcatta     240

actgaaactc atgcaattct tcaacgtatg cgtgaactta ctgttcaagc aggaaacaca     300

ggtacacaac aagctgaaga tcttggtgca attaaagatg aaatggatgc gcttatcgag     360

gaaattgatg gcatttcaaa ccgtactgaa tttaacggta aaaagttgct agacggaact     420

aattctactg atggtttcac attccaaatt ggtgcgaatg ctggccaaca actaaatgta     480

aaaattgaca gcatgtcatc aactgcttta ggagtaaacg cacttgatgt aacagatttc     540

gctgctactg cttttgatga tcaacttaaa agtattgata ctgccatcaa tactgtatca     600

actcaacgtg ctaaattagg tgcggtacaa aaccgtctag agcatacaat caacaactta     660

ggcgcttctg gtgaaaacct gacagctgct gagtctcgta tccgtgacgt tgacatggct     720

aaagaaatga gcgagttcac taagaacaac attctttctc aagcttctca agctatgctt     780

gctcaagcta accaacagcc tcaaaacgta cttcaattat tacgtggtgg cggagggtct     840

accatggtta gatctggttg taagccttgc atatgtacag tcccagaagt atcatctgtc     900

ttcatcttcc ccccaaagcc caaggatgtg ctcaccatta ctctgactcc taaggtcacg     960

tgtgttgtgg tagacatcag caaggatgat cccgaggtcc agttcagctg gtttgtagat    1020

gatgtggagg tgcacacagc tcagacgcaa ccccgggagg agcagttcaa cagcactttc    1080

cgctcagtca gtgaacttcc catcatgcac caggactggc tcaatggcaa ggagttcaaa    1140

tgcagggtca acagtgcagc tttccctgcc cccatcgaga aaaccatctc caaaaccaaa    1200

ggcagaccga aggctccaca ggtgtacacc attccacctc ccaaggagca gatggccaag    1260

gataaagtca gtctgacctg catgataaca gacttcttcc ctgaagacat tactgtggag    1320

tggcagtgga atgggcagcc agcggagaac tacaagaaca ctcagcccat catggacaca    1380

gatggctctt acttcgtcta cagcaagctc aatgtgcaga agagcaactg ggaggcagga    1440

aatactttca cctgctctgt gttacatgag ggcctgcaca accaccatac tgagaagagc    1500

ctctcccact ctcctggtaa atga                                           1524


<210>    19
<211>    1554
<212>    DNA
<213>    Artificial Sequence

<220>
<223>    polynucleotide encoding mIgG1-Fc-Bsflagellin  (with linker 2)

<400>    19
atgagaatta accacaatat tgcagcactt aacacattga atcgtttggg ttcaaacaac      60

ggagcagcac aaaagaatat ggagaagctt tcttcaggtc ttcgtatcaa ccgtgcggga     120

gatgacgcag caggtctagc gatctctgaa aaaatgagag gacaaatcag aggtcttgaa     180

atggcttcta aaaactctca agatggaatc tctcttatcc aaacagctga gggtgcatta     240

actgaaactc atgcaattct tcaacgtatg cgtgaactta ctgttcaagc aggaaacaca     300

ggtacacaac aagctgaaga tcttggtgca attaaagatg aaatggatgc gcttatcgag     360

gaaattgatg gcatttcaaa ccgtactgaa tttaacggta aaaagttgct agacggaact     420

aattctactg atggtttcac attccaaatt ggtgcgaatg ctggccaaca actaaatgta     480

aaaattgaca gcatgtcatc aactgcttta ggagtaaacg cacttgatgt aacagatttc     540

gctgctactg cttttgatga tcaacttaaa agtattgata ctgccatcaa tactgtatca     600

actcaacgtg ctaaattagg tgcggtacaa aaccgtctag agcatacaat caacaactta     660

ggcgcttctg gtgaaaacct gacagctgct gagtctcgta tccgtgacgt tgacatggct     720

aaagaaatga gcgagttcac taagaacaac attctttctc aagcttctca agctatgctt     780

gctcaagcta accaacagcc tcaaaacgta cttcaattat tacgtggtgg cggagggtct     840

ggtggcggag ggtctggtgg cggagggtct accatggtta gatctggttg taagccttgc     900

atatgtacag tcccagaagt atcatctgtc ttcatcttcc ccccaaagcc caaggatgtg     960

ctcaccatta ctctgactcc taaggtcacg tgtgttgtgg tagacatcag caaggatgat    1020

cccgaggtcc agttcagctg gtttgtagat gatgtggagg tgcacacagc tcagacgcaa    1080

ccccgggagg agcagttcaa cagcactttc cgctcagtca gtgaacttcc catcatgcac    1140

caggactggc tcaatggcaa ggagttcaaa tgcagggtca acagtgcagc tttccctgcc    1200

cccatcgaga aaaccatctc caaaaccaaa ggcagaccga aggctccaca ggtgtacacc    1260

attccacctc ccaaggagca gatggccaag gataaagtca gtctgacctg catgataaca    1320

gacttcttcc ctgaagacat tactgtggag tggcagtgga atgggcagcc agcggagaac    1380

tacaagaaca ctcagcccat catggacaca gatggctctt acttcgtcta cagcaagctc    1440

aatgtgcaga agagcaactg ggaggcagga aatactttca cctgctctgt gttacatgag    1500

ggcctgcaca accaccatac tgagaagagc ctctcccact ctcctggtaa atga          1554

<210>    20
<211>    2286
<212>    DNA
<213>    Artificial Sequence

<220>
<223>    polynucleotide encoding hIgG4-Fc-Sdflagellin (with linker 1)

<400>    20
atgtacagga tgcaactcct gtcttgcatt gcactaagtc ttgcacttgt cacgaattcg      60

atatcgatgg cacaagtcat taatacaaac agcctgtcgc tgttgaccca gaataacctg     120

aacaaatctc agtcctcact gagttccgct attgagcgtc tgtcctctgg tctgcgtatc     180

aacagcgcga aagacgatgc ggcaggccag gcgattgcta accgcttcac ttctaatatc     240

aaaggtctga ctcaggcttc ccgtaacgct aacgacggca tttctattgc gcagaccact     300

gaaggtgcgc tgaatgaaat caacaacaac ctgcagcgtg tgcgtgagtt gtctgttcag     360

gccactaacg ggactaactc tgattccgat ctgaaatcta tccaggatga aattcagcaa     420

cgtctggaag aaatcgatcg cgtttctaat cagactcaat ttaacggtgt taaagtcctg     480

tctcaggaca accagatgaa aatccaggtt ggtgctaacg atggtgaaac cattaccatc     540

gatctgcaaa aaattgatgt gaaaagcctt ggccttgatg ggttcaatgt taatgggcca     600

aaagaagcga cagtgggtga tctgaaatcc agcttcaaga atgttacggg ttacgacacc     660

tatgcagcgg gtgccgataa atatcgtgta gatattaatt ccggtgctgt agtgactgat     720

gcagtagcac cggataaagt atatgtaaat gcagcaaacg gtcagttaac aactgacgat     780

gcggaaaata acactgcggt tgatctcttt aagaccacta aatctactgc tggtaccgct     840

gaagccaaag cgatagctgg tgccattaaa ggtggtaagg aaggagatac ctttgattat     900

aaaggcgtga cttttactat tgatacaaaa actggtgatg acggtaatgg taaggtttct     960

actaccatca atggtgaaaa agttacgtta actgtcgctg atattgccat tggcgcggcg    1020

gatgttaatg ctgctacctt acaatcaagc aaaaatgttt atacatctgt agtgaacggt    1080

cagtttactt ttgatgataa aaccaaaaac gagagtgcga aactttctga tttggaagca    1140

aacaatgctg ttaagggcga aagtaaaatt acagtaaatg gggctgaata tactgctaac    1200

gccacgggtg ataagatcac cttagctggc aaaaccatgt ttattgataa aacagcttct    1260

ggcgtaagta cattaatcaa tgaagacgct gccgcagcca agaaaagtac cgctaaccca    1320

ctggcttcaa ttgattctgc attgtcaaaa gtggacgcag ttcgttcttc tctgggggca    1380

attcaaaacc gttttgattc agccattacc aaccttggca atacggtaac caatctgaac    1440

tccgcgcgta gccgtatcga agatgctgac tatgcaacgg aagtttctaa tatgtctaaa    1500

gcgcagattc tgcagcaggc tggtacttcc gttctggcgc aggctaacca ggttccgcaa    1560

aacgtcctct ctttactgcg tggtggcgga gggtctgcca tggttagatc tcccccatgc    1620

ccatcatgcc cagcacctga gttcctgggg ggaccatcag tcttcctgtt ccccccaaaa    1680

cccaaggaca ctctcatgat ctcccggacc cctgaggtca cgtgcgtggt ggtggacgtg    1740

agccaggaag accccgaggt ccagttcaac tggtacgtgg atggcgtgga ggtgcataat    1800

gccaagacaa agccgcggga ggagcagttc aacagcacgt accgtgtggt cagcgtcctc 1860

accgtcctgc accaggactg gctgaacggc aaggagtaca agtgcaaggt ctccaacaaa 1920

ggcctcccgt cctccatcga gaaaaccatc tccaaagcca aagggcagcc ccgagagcca 1980

caggtgtaca ccctgccccc atcccaggag gagatgacca agaaccaggt cagcctgacc 2040

tgcctggtca aaggcttcta ccccagcgac atcgccgtgg agtgggagag caatgggcag 2100

ccggagaaca actacaagac cacgcctccc gtgctggact ccgacggctc cttcttcctc 2160

tacagcaggc taaccgtgga caagagcagg tggcaggagg ggaatgtctt ctcatgctcc 2220

gtgatgcatg aggctctgca caaccactac acacagaaga gcctctccct gtctctgggt 2280

aaataa 2286

<210> 21
<211> 1932
<212> DNA
<213> Artificial Sequence

<220>
<223> polynucleotide encoding hIgG4-Fc-Paflagellin (with linker 1)

<400> 21
atgtacagga tgcaactcct gtcttgcatt gcactaagtc ttgcacttgt cacgaattcg 60

atatcgatgg ccttgaccgt caacaccaac atcgcttcgc tgaacactca gcggaaccag 120

aacaactctt ccgcgtcgct gaacacttcg ctgcagcgtc tgtccaccgg ttcgcgcatc 180

aacagcgcca aggacgacgc cgccggcctg cagatcgcca accgtctgac cagccaggtc 240

aacggcctga acgtggctac caagaacgcc aacgatggta tctccctggc gcagaccgct 300

gaaggcgccc tgcagcagtc gaccaacatc ctgcagcgta tgcgtgacct gtccctgcag 360

tcggccaacg gctccaacag cgactccgag cgtaccgctc tgaatggcga agtgaagcaa 420

ctgcagaaag aactggatcg tatcagcaac accaccacct tcggtggccg caagctgctc 480

gacggttcct tcggcgtcgc cagcttccag gtgggttcgg ccgccaacga aatcatcagc 540

gtcggcatcg acgagatgag cgcagagtcg ctgaacggca cctacttcaa ggctgacggc 600

ggcggcgcgg tcactgctgc aaccgcttcg ggcaccgtcg acatcgcgat cgacatcacc 660

ggcggcagcg ccgtgaacgt caaggtcgac atgaagggca acgaaaccgc cgagcaggcg 720

gctgccaaga tcgccgctgc ggtcaacgac gccaacgtcg gcatcggtgc cttcaccgac 780

ggcgcgcaga tcagctacgt gtccaaggct agtgcggatg gtactaccag tgcggtttcc 840

ggcgtagcca tcaccgacac cggcagcacc ggcgcaggca ccgcggcagg caccacgacg 900

ttcaccgagg ctaacgacac cgtcgccaag atcgacatct ccaccgcgaa gggcgctcag 960

tccgccgtgc tggtgatcga cgaggcgatc aagcagatcg acgcccagcg tgccgacctc 1020

ggtgcggtgc agaaccgctt cgacaacacc ataaacaacc tgaagaacat cggtgagaac 1080

gtatcggctg ctcgcggccg gatcgaagac accgacttcg cagccgaaac cgccaacctg 1140

accaagaacc aagtgctgca acaagccggc accgcgatcc tggcccaggc caaccagctg 1200

ccgcagtcgg ttctgagcct gctgcgcggt ggcggagggt ctgccatggt tagatctccc 1260

ccatgcccat catgcccagc acctgagttc ctggggggac catcagtctt cctgttcccc 1320

ccaaaaccca aggacactct catgatctcc cggacccctg aggtcacgtg cgtggtggtg 1380

gacgtgagcc aggaagaccc cgaggtccag ttcaactggt acgtggatgg cgtggaggtg 1440

cataatgcca agacaaagcc gcgggaggag cagttcaaca gcacgtaccg tgtggtcagc 1500

gtcctcaccg tcctgcacca ggactggctg aacggcaagg agtacaagtg caaggtctcc 1560

aacaaaggcc tcccgtcctc catcgagaaa accatctcca aagccaaagg gcagccccga 1620

gagccacagg tgtacaccct gcccccatcc caggaggaga tgaccaagaa ccaggtcagc 1680

ctgacctgcc tggtcaaagg cttctacccc agcgacatcg ccgtggagtg ggagagcaat 1740

gggcagccgg agaacaacta caagaccacg cctcccgtgc tggactccga cggctccttc 1800

ttcctctaca gcaggctaac cgtggacaag agcaggtggc aggaggggaa tgtcttctca 1860

tgctccgtga tgcatgaggc tctgcacaac cactacacac agaagagcct ctccctgtct 1920

ctgggtaaat aa 1932

<210> 22
<211> 2421
<212> DNA
<213> Artificial Sequence

<220>
<223> polynucleotide encoding hIgG4-Fc-Sfflagellin (with linker 1)

<400> 22
atgtacagga tgcaactcct gtcttgcatt gcactaagtc ttgcacttgt cacgaattcg 60

atatcgatgg cacaagtcat taataccaac agcctctcgc tgatcactca aaataatatc 120

aacaagaacc agtctgcgct gtcgagttct atcgagcgtc tgtcttctgg cttgcgtatt 180

aacagcgcga aggatgacgc cgcgggtcag gcgattgcta accgttttac ttctaacatt 240

aaaggcctga ctcaggctgc acgtaacgcc aacgacggta tttctgttgc acagaccact 300

gaaggcgcgc tgtccgaaat caacaacaac ttacagcgta tccgtgagct gacggttcag 360

gcttctaccg ggactaactc tgattcggat ctggactcca ttcaggacga aatcaaatcc 420

cgtctcgacg aaattgaccg cgtatccggt cagacccagt tcaacggcgt gaacgtactg 480

gcaaaagacg gttcgatgaa aattcaggtt ggtgcgaatg acggccagac tatcactatt 540

gatctgaaga aaattgactc tgatacgctg gggctgaatg ggtttaatgt gaatggtggc 600

ggggctgtgg ctaatactgc tgcatctaaa gctgacttgg tagctgctaa tgcaacagtg 660

gtcggcaaca aatatactgt gagtgcgggt tacgatgctg ctaaagcgtc tgatttgctg 720

gctggagtta gtgatggtga tactgttcag gcaaccatta ataacggctt cggaacggcg 780

gctagtgcaa cgaattacaa gtatgacagt gcaagtaagt catactcttt tgataccaca 840

acggcttcag ctgccgatgt tcagaaatat ttgaccccgg gagttggtga taccgctaag 900

ggcactatta ctatcgatgg ttctgcacag gatgttcaga tcagcagtga tggtaaaatt 960

acggcaagca atggagataa actctacatt gatacaactg ggcgcttaac gaaaaacggc 1020

tctggtgctt ctttgactga ggctagtctg tccacacttg cagccaataa taccaaagcg 1080

acaaccattg acattggcgg tacctctatc tcctttaccg gtaatagtac tacgccggac 1140

actattactt attcagtaac aggtgcaaaa gttgatcagg cagctttcga taaagctgta 1200

tcaacctcgg gaaacaatgt tgatttcact actgcaggtt atagcgtcaa cggcacaact 1260

ggcgctgtaa caaaaggtgt tgattcggtt tatgttgata acaacgaggc gttgaccaca 1320

tctgatactg tagattttta cctacaggat gatggttcag tgactaacgg cagcggtaag 1380

gcagtttata aagatgctga tggtaaattg acgacagatg ctgaaacaaa agcagctacg 1440

acagccgatc ccctgaaagc tctggatgaa gccatcagct ccatcgacaa attccgctcc 1500

tccctgggtg cggtgcaaaa ccgtttggat tccgcagtca ccaacctgaa caacaccact 1560

accaacctgt ctgaagcgca gtcccgtatt caggacgccg actatgcgac cgaagtgtcc 1620

aacatgtcga aagcgcagat tatccagcag gccggtaact ccgtgctggc aaaagccaac 1680

caggtaccgc agcaggttct gtctctgctg cagggtggtg gcggagggtc tgccatggtt 1740

agatctcccc catgcccatc atgcccagca cctgagttcc tggggggacc atcagtcttc 1800

ctgttccccc caaaacccaa ggacactctc atgatctccc ggacccctga ggtcacgtgc 1860

gtggtggtgg acgtgagcca ggaagacccc gaggtccagt tcaactggta cgtggatggc 1920

gtggaggtgc ataatgccaa gacaaagccg cgggaggagc agttcaacag cacgtaccgt 1980

gtggtcagcg tcctcaccgt cctgcaccag gactggctga acggcaagga gtacaagtgc 2040

aaggtctcca acaaaggcct cccgtcctcc atcgagaaaa ccatctccaa agccaaaggg 2100

cagccccgag agccacaggt gtacaccctg cccccatccc aggaggagat gaccaagaac 2160

caggtcagcc tgacctgcct ggtcaaaggc ttctacccca gcgacatcgc cgtggagtgg 2220

gagagcaatg ggcagccgga gaacaactac aagaccacgc ctcccgtgct ggactccgac 2280

ggctccttct tcctctacag caggctaacc gtggacaaga gcaggtggca ggaggggaat 2340

gtcttctcat gctccgtgat gcatgaggct ctgcacaacc actacacaca gaagagcctc 2400

tccctgtctc tgggtaaata a 2421

<210>    23
<211>    2265
<212>    DNA
<213>    Artificial Sequence

<220>
<223>    polynucleotide encoding hIgG4-Fc-Ecflagellin (with linker 1)

<400>    23
atgtacagga tgcaactcct gtcttgcatt gcactaagtc ttgcacttgt cacgaattcg      60

atatcgatgg cacaagtcat taataccaac agcctctcgc tgatcactca aaataatatc     120

aacaagaacc agtctgcgct gtcgagttct atcgagcgtc tgtcttctgg cttgcgtatt     180

aacagcgcga aggatgacgc agcgggtcag gcgattgcta accgtttcac ctctaacatt     240

aaaggcctga ctcaggcggc ccgtaacgcc aacgacggta tctccgttgc gcagaccacc     300

gaaggcgcgc tgtccgaaat caacaacaac ttacagcgtg tgcgtgaact gacggtacag     360

gccactaccg gtactaactc tgagtctgat ctgtcttcta tccaggacga aattaaatcc     420

cgtctggatg aaattgaccg cgtatctggt cagacccagt tcaacggcgt gaacgtgctg     480

gcaaaaaatg gctccatgaa aatccaggtt ggcgcaaatg ataaccagac tatcactatc     540

gatctgaagc agattgatgc taaaactctt ggccttgatg gttttagcgt taaaaataac     600

gatacagtta ccactagtgc tccagtaact gcttttggtg ctaccaccac aaacaatatt     660

aaacttactg gaattaccct ttctacggaa gcagccactg atactggcgg aactaaccca     720

gcttcaattg agggtgttta tactgataat ggtaatgatt actatgcgaa aatcaccggt     780

ggtgataacg atgggaagta ttacgcagta acagttgcta atgatggtac agtgacaatg     840

gcgactggag caacggcaaa tgcaactgta actgatgcaa atactactaa agctacaact     900

atcacttcag gcggtacacc tgttcagatt gataatactg caggttccgc aactgccaac     960

cttggtgctg ttagcttagt aaaactgcag gattccaagg gtaatgatac cgatacatat    1020

gcgcttaaag atacaaatgg caatctttac gctgcggatg tgaatgaaac tactggtgct    1080

gtttctgtta aaactattac ctatactgac tcttccggtg ccgccagttc tccaaccgcg    1140

gtcaaactgg gcggagatga tggcaaaaca gaagtggtcg atattgatgg taaaacatac    1200

gattctgccg atttaaatgg cggtaatctg caaacaggtt tgactgctgg tggtgaggct    1260

ctgactgctg ttgcaaatgg taaaaccacg gatccgctga aagcgctgga cgatgctatc    1320

gcatctgtag acaaattccg ttcttccctc ggtgcggtgc aaaaccgtct ggattccgcg    1380

gttaccaacc tgaacaacac cactaccaac ctgtctgaag cgcagtcccg tattcaggac    1440

gccgactatg cgaccgaagt gtccaatatg tcgaaagcgc agatcatcca gcaggccggt    1500

aactccgtgt tggcaaaagc taaccaggta ccgcagcagg ttctgtctct gctgcagggt    1560

ggtggcggag ggtctgccat ggttagatct cccccatgcc catcatgccc agcacctgag    1620

ttcctggggg gaccatcagt cttcctgttc cccccaaaac ccaaggacac tctcatgatc 1680

tcccggaccc ctgaggtcac gtgcgtggtg gtggacgtga gccaggaaga ccccgaggtc 1740

cagttcaact ggtacgtgga tggcgtggag gtgcataatg ccaagacaaa gccgcgggag 1800

gagcagttca acagcacgta ccgtgtggtc agcgtcctca ccgtcctgca ccaggactgg 1860

ctgaacggca aggagtacaa gtgcaaggtc tccaacaaag gcctcccgtc ctccatcgag 1920

aaaaccatct ccaaagccaa agggcagccc cgagagccac aggtgtacac cctgccccca 1980

tcccaggagg agatgaccaa gaaccaggtc agcctgacct gcctggtcaa aggcttctac 2040

cccagcgaca tcgccgtgga gtgggagagc aatgggcagc cggagaacaa ctacaagacc 2100

acgcctcccg tgctggactc cgacggctcc ttcttcctct acagcaggct aaccgtggac 2160

aagagcaggt ggcaggaggg gaatgtcttc tcatgctccg tgatgcatga ggctctgcac 2220

aaccactaca cacagaagag cctctccctg tctctgggta aataa 2265

<210> 24
<211> 43
<212> DNA
<213> Artificial Sequence

<220>
<223> Ecflagellin-hIgG4-Fc2-5′ EcoRV-G

<400> 24
ccggatatcg atggcacaag tcattaatac caacagcctc tcg 43

<210> 25
<211> 48
<212> DNA
<213> Artificial Sequence

<220>
<223> Ecflagellin-(GGGGS)-hIgG1-Fc2-3′ NcoI-C

<400> 25
gaccatggca gaccctccgc caccctgcag cagagacaga acctgctg 48

<210> 26
<211> 43
<212> DNA
<213> Artificial Sequence

<220>
<223> Paflagellin-hIgG4-Fc2-5′ EcoRV-G

<400> 26
ccggatatcg atggccttga ccgtcaacac caacatcgct tcg 43

<210> 27
<211> 51
<212> DNA
<213> Artificial Sequence

<220>
<223> Ecflagellin-(GGGGS)-hIgG1-Fc2-3' NcoI-C

<400> 27
gaccatggca gaccctccgc caccgcgcag caggctcaga accgactgcg g 51

<210> 28
<211> 37
<212> DNA
<213> Artificial Sequence

<220>
<223> Sdflagellin-hIgG4-Fc2-5' EcoRV-G

<400> 28
ccggatatcg atggcacaag tcattaatac aaacagc 37

<210> 29
<211> 48
<212> DNA
<213> Artificial Sequence

<220>
<223> Sdflagellin-(GGGGS)-hIgG1-Fc2-3' NcoI-C

<400> 29
gaccatggca gaccctccgc caccacgcag taaagagagg acgttttg 48

<210> 30
<211> 37
<212> DNA
<213> Artificial Sequence

<220>
<223> Sfflagellin-hIgG4-Fc2-5' EcoRV-G

<400> 30
ccggatatcg atggcacaag tcattaatac caacagc 37

<210> 31
<211> 45
<212> DNA
<213> Artificial Sequence

<220>
<223> Sfflagellin-(GGGGS)-hIgG1-Fc2-3' NcoI-C

<400> 31
gaccatggca gaccctccgc caccaccctg cagcagagac agaac 45

<210>    32
<211>    43
<212>    DNA
<213>    Artificial Sequence

<220>
<223>    CTGBsflagellin-hIgG4-Fc2-5′ EcoRV-G


<400>    32
ccggatatcg atgagaatta accacaatat tgcagcactt aac                43


<210>    33
<211>    54
<212>    DNA
<213>    Artificial Sequence

<220>
<223>    Bsflagellin-(GGGGS)-hIgG1-Fc2-3′ NcoI-C


<400>    33
gaccatggca gaccctccgc caccacgtaa taattgaagt acgttttgag gctg    54


<210>    34
<211>    42
<212>    DNA
<213>    Artificial Sequence

<220>
<223>    pET49b-bsflagellin vector forward primer


<400>    34
ccggaattca tgagaattaa ccacaatatt gcagcactta ac                 42


<210>    35
<211>    54
<212>    DNA
<213>    Artificial Sequence

<220>
<223>    pET49b-bsflagellin vector reverse primer - 1


<400>    35
gaccatggta gaccctccgc caccacgtaa taattgaagt acgttttgag gctg    54


<210>    36
<211>    84
<212>    DNA
<213>    Artificial Sequence

<220>
<223>    pET49b-bsflagellin vector reverse primer - 2

<400>    36
gaccatggta gaccctccgc caccagaccc tccgccacca gaccctccgc caccacgtaa          60

taattgaagt acgttttgag gctg                                                  84

## Description

### THECHNICAL FIELD

This application claims priority to Korean Patent Application No. 10-2019-0046866 filed on April 22, 2019, and the entire specifications of which are incorporated herein by reference in their entireties.

The present invention relates to a flagellin fusion proteins and a use thereof and, more specifically, to a fusion protein comprising flagellin, a fragment thereof; or a variant thereof; and an immunoglobulin Fc region and use in which a toll-like receptor 5 (TLR5) stimulating activity thereof is used.

### BACKGROUND OF THE INVENTION

Flagella are an important component that determines the motility of bacteria and is largely composed of a hook, a basal body, and a filament. It is also known that flagella have a function of determining bacterial swimming or swarming motility, bacterial taxis, and forming a biofilm to determine the ability of pathogenic microorganisms to adhere. The structural unit protein constituting the filament of the flagella is called flagellin, and the flagellin is regularly assembled to form the filament. Hayashi *et al.* reported that mammalian-expressed TLR5 recognizes flagellin from Gram-negative and Gram-positive bacteria and activates NF-κB(Hayashi F, Smith KD, OzinskyA, Hawn TR, Yi EC, Goodlett DR, Eng JK, Akira S, Underhill DM, Aderem A: Nature 410:1099-1103, 2001).

Flagellin is a structural protein that assembles into the whip-stalk-like filaments of bacterial flagella, which extend from the cell surface and function to allow bacteria to move. Flagellin promotes the penetration and invasion of pathogenic bacteria into host cells by acting as a virulence factor. Because flagellin is found exclusively in bacteria and is one of the most abundant proteins in flagellated bacteria, flagellin is a major target for host immune surveillance. Upon bacterial invasion, flagellin activates innate immunity, which is detected by Toll-like receptor 5 (TLR5) and NAIP5/NLRC4 in the host and contributes to the immediate clearance of pathogens from the host.

TLR5 is an innate immune receptor located on the cell surface and consists of an extracellular leucine-rich repeat (LRR) domain, a transmembrane domain and an intracellular domain. TLR5 recognizes flagellin as a pathogen-associated molecular pattern using an extracellular domain and activates the MyD88-dependent signaling pathway and NF-κB-mediated inflammatory cytokine production.

Flagellin has been of interest as a target for the development of vaccine carrier proteins or vaccine adjuvants because it serves as the first line of defense against flagellate-pathogenic bacteria. A fusion protein of antigen and flagellin has been demonstrated to be effective as an experimental vaccine against a variety of communicable diseases including West Nile fever, malaria, infectious diseases and tuberculosis, and it has been reported that flagellin-induced TLR5 activation also protects hematopoietic cells and radiation-induced gastrointestinal tissue and affects the survival and growth of cancer cells.

Flagellin contains two to four domains. For example, the *Bacillus subtilis* Hag flagella, *Pseudomonas aeruginosa* type A FliC flagella, *Salmonella enterica subspecies enterica serovar Typhimurium* FliC flagellin each contain 2 (D0 and D1), 3 (D0, D1, and D2) and 4 (D0, D1, D2, and D3) domains. The D0 and D1 domains common in these are located at the center of flagellar filaments by mediating inter-flagellar interactions and are highly conserved among bacterial species due to the functional importance of filament formation. Since flagellin monomer, not a polymerized filament, activates TLR5, it is thought that the aforementioned D0 and D1 domains may be major stimulators of TLR5. In the 3- and 4-domain flagellins, the D1 domain extends to auxiliary domains (D2 and D3) on the surface of the flagellar filaments, contributing little or no to filament formation. Unlike the D0 and D1 domains, the D2 or D3 domains exhibit substantial changes in sequence and structure, and are believed to activate adaptive immunity and induce undesirable toxicity in flagellin-based therapies. Therefore, the radiation therapy bio-drug CBLB502 containing D0/D1 was developed by removing the hypervariable region (D2 and D3 domains) from *Salmonella* flagellin.

Many Gram-positive bacteria, such as *Bacillus subtilis* and *Clostridium difficile,* express flagellin deficient in the hypervariable region, so flagellar filaments contain the minimum regions (D0 and D1 regions) required for TLR5 activation and flagellin polymerization.

Flagellin-TLR5 interaction and its cellular consequences have been extensively studied using *Salmonella flagellin.* Structural and biochemical studies of the complex *between Salmonella enterica subspecies enterica serovar Dublin* flagellin D1-D2 region(sdflagellin D1-D2) and the N-terminal fragment of zebrafish TLR5 is known that flagellin and TLR5 form a 1:1 complex through 'primary bond', afterwards, to be homodimerized into a 2:2 complex through 'second dimerization'.

Based on the results of many studies on the interaction between flagellin and TLR5, various studies are being conducted to enhance TLR5 activation through the modification of flagellin, but there is no research on a new type of flagellin protein targeting the flagellin-TLR5 2:2 complex structure.

### DETAILED DESCRIPTION OF THE INVENTION

### TECHNICAL PROBLEM

Accordingly, the present inventors focused on the formation of a 2:2 complex structure when flagellin activates TLR5, and as a result of repeated studies to develop a new type of protein with improved TLR5 activation ability, it was discovered that a new type of fusion protein in which flagellin and immunoglobulin Fc are fused exhibits significantly superior TLR5 activation ability compared to wild-type flagellin, known flagellin fragments, and the like, and completed the present invention.

Accordingly, an object of the present invention is to provide a fusion protein comprising a flagellin, a fragment thereof, or a variant thereof; and an immunoglobulin Fc region.

Another object of the present invention is to provide a polynucleotide encoding the fusion protein.

Another object of the present invention is to provide a vector comprising the polynucleotide.

Another object of the present invention is to provide a transformant transformed with the vector.

Another object of the present invention is to provide a pharmaceutical composition comprising the fusion protein as an active ingredient.

It is also to provide a pharmaceutical composition consisting of the fusion protein.

It is also to provide a pharmaceutical composition essentially consisting of the fusion protein.

Another object of the present invention is to provide a vaccine adjuvant comprising the fusion protein as an active ingredient.

It is also to provide a vaccine adjuvant consisting of the fusion protein.

It is also to provide a vaccine adjuvant essentially consisting of the fusion protein.

Another object of the present invention is to provide use of the fusion protein for the preparation of a pharmaceutical agent for the treatment of injury by radiation exposure; the treatment of reperfusion injury; the treatment of inflammatory bowel disease; the treatment of autoimmune disease; the treatment of viral infection; the treatment of metabolic disease; the treatment of aging; the enhancement of immune function; or the treatment of cancer.

Another object of the present invention is to provide a method for treating injury by exposure to radiation; treating reperfusion injury; treating inflammatory bowel disease; treating autoimmune disease, treating viral infection; treating metabolic disease; treating aging; enhancing immune function; or treating cancer, comprising administering to a subject in need thereof an effective amount of a composition comprising the fusion protein as an active ingredient.

### TECHNICAL SOLUTION

In order to achieve the above object of the present invention, the present invention provides a flagellin, a fragment thereof or a variant thereof; and an immunoglobulin Fc region.

In order to achieve another object of the present invention, the present invention provides a polynucleotide encoding the fusion protein.

In order to achieve another object of the present invention, the present invention provides a vector comprising the polynucleotide

In order to achieve another object of the present invention, the present invention provides a transformant transformed with the vector

In order to achieve another object of the present invention, the present invention provides a pharmaceutical composition comprising the fusion protein as an active ingredient.

The present invention also provides a pharmaceutical composition consisting of the fusion protein.

The present invention also provides a pharmaceutical composition essentially consisting of the fusion protein.

In order to achieve another object of the present invention, the present invention provides a vaccine adjuvant comprising the fusion protein as an active ingredient.

The present invention also provides a vaccine adjuvant consisting of the fusion protein.

The present invention also provides a vaccine adjuvant essentially consisting of the fusion protein.

In order to achieve another object of the present invention, the present invention provides use of the fusion protein for the preparation of a pharmaceutical agent for the treatment of injury by radiation exposure; the treatment of reperfusion injury; the treatment of inflammatory bowel disease; the treatment of autoimmune disease; the treatment of viral infection; the treatment of metabolic disease; the treatment of aging; the enhancement of immune function; or the treatment of cancer.

In order to achieve another object of the present invention, the present invention provides a method for treating injury by exposure to radiation; treating reperfusion injury; treating inflammatory bowel disease; treating autoimmune disease, treating viral infection; treating metabolic disease; treating aging; enhancing immune function; or treating cancer, comprising administering to a subject in need thereof an effective amount of a composition comprising the fusion protein as an active ingredient.

Hereinafter, the present invention will be described in detail.

The present invention provides a flagellin, a fragment thereof or a variant thereof; and an immunoglobulin Fc region.

In the present invention, the flagellin may induce an immune response in an infected host when the flagellate bacterium is infected. More specifically, Toll-like receptor 5 (TLR5) present on the cell membrane surface of the human body induces intracellular signal transduction through interaction with the flagellin, and through this, the expression of NF-kB, a transcription factor, is increased to induce activation of innate immune signals, as well as regulate acquired immune responses.

Flagellin proteins are described well, for example, in US Pat. No. 6,585,980, No. 6,130,082; No. 5,888,810; No. 5,618,533; No. 4,886,748 and US Patent Publication No. US2003/0044429 A1; and Donnelly et al. (2002) J. Biol. Chem. 43:4045 and the like. Most Gram-negative bacteria express flagella, a surface structure that provides motility. A flagellum consists of a basal body, a filament, and a hook connecting the two. The filaments consist of a long polymer of flagellin, a single protein, with a small cap protein formed at the tip.

Polymerization of flagellin is mediated by regions conserved at the N- and C-terminus, whereas the intermediate hypervariable region of flagellin protein is highly variable in sequence and length between species.

In the present invention, the flagellin may be flagellin derived from any suitable bacteria. A number of flagellin genes have been cloned and sequenced in the art and may be referred to.

In the present invention, the non-limiting source of the flagellin is *Bacillus* genus, *Salmonella* genus *Helicobacter* genus, *Vibrio* genus, *Serratia* genus, *Shigella* genus, *Treponema* genus, *Legionella* genus, *Borrelia* genus, *Clostridium* genus, *Agrobacterium* genus, *Bartonella* genus, *Proteus* genus, *Pseudomonas* genus, *Escherichia* genus, *Listeria* genus, *Yersinia* genus, *Campylobacter* genus, *Roseburia* genus or *Marinobacter* genus microorganisms, preferably is *Bacillus* genus, *Salmonella* genus or *Vibrio* genus microorganisms.

More preferably, in the present invention, the flagellin may be derived from *Salmonella enteritidis, Salmonella typhimurium, Salmonella Dublin, Salmonella enterica, Helicobacter pylori, Vibrio cholera, Vibrio vulnificus, Vibrio fibrisolvens, Serratia marcesens, Shigella flexneri, Treponema pallidum, Borrelia burgdorferei, Clostridium difficile, Agrobacterium tumefaciens, Bartonella clarridgeiae, Proteus mirabilis, Bacillus subtilis, Bacillus cereus, Bacillus halodurans, Pseudomonas aeruginosa, Escherichia coli, Listeria monocytogenes, Yersinia pestis, Campylobacter spp, Roseburia spp* or *Marinobacter spp.*

Even more preferably, in the present invention, the flagellin may be derived from *Salmonella enteritidis, Salmonella typhimurium, Salmonella Dublin, Salmonella enterica, Vibrio cholera, Vibrio vulnificus, Vibrio fibrisolvens, Bacillus subtilis, Bacillus cereus* or *Bacillus halodurans,*

Most preferably, it may be flagellin derived from *Bacillus subtilis.*

TLR5 recognizes the evolutionarily conserved region of bacterial flagellin required for flagellar filament assembly and movement, but some α and ε Proteobacteria(*Campylobacter jejuni, Helicobacter pylori,* and *Bartonella bacilliformis*) flagellins are not recognized by TLR5, as reported in the following papers. [(2003) Microbes Infect. 5, 1345-1356.; J. Infect. Dis. 189, 1914-1920.; (2002) Nat. Rev. Cancer 2, 28-37; (2001) Clin. Infect. Dis. 32, 1201-1206. pmid: 11283810]. Bacterial flagellins recognized by TLR5 have similarly conserved major amino acid sequences in the D0 and D1 domains, and bacterial flagellins that are not recognized by TLR5 have different amino acid sequences(PNAS June 28, 2005 102 (26) 9247-9252; Scientific Reports | 7:40878 | DOI: 10.1038/srep40878). Accordingly, in the present invention, the flagellin may be flagellin including a conserved sequence recognized by TLR5 in the D0 and D1 domains.

In one embodiment of the present invention, with reference to previously reported papers, flagellin of five representative bacteria in which a common amino acid sequence recognized by TLR5 is conserved in the D0 and D1 domains was prepared as an Fc-fusion protein.

The N-terminal and C-terminal constant regions of flagellin are well-characterized in the art, and for example, reference can be made to the literature[Mimori-Kiyosue et al., (1997) J. Mol. Byrol. 270: 222-237; lino et al., (1977) Ann. Genet. 11:161-182; and Schoenhals et al. (1993) J. Bacteriol. 175: 5395-5402]. As will be well understood by those skilled in the art, the size of the constant region may vary somewhat depending on the source of the flagellin protein. In general, an N-terminal constant domain comprises about 170 or 180 N-terminal amino acids of a protein, whereas a C-terminal constant domain typically comprises about 85-100 C-terminal amino acids. The central hypervariable region varies considerably depending on the size and order among bacteria, and most of the difference in molecular mass can be explained by the hypervariable region. The N-and C-terminal constant regions of flagellin proteins from various bacteria are known, and flagellin derived from bacteria, which is not yet known, can also be easily determined by a person skilled in the art using techniques known in the art to determine the crystal structure of flagellin monomer.

As used herein, the terms "flagellin", "flagellin N-terminal constant region" and "flagellin C-terminal constant region" include flagellin active fragments and variants derived from any of the above exemplified bacteria. In addition, wild-type flagellin or portions of flagellin may be modified to increase safety and/or immune response and/or as a result of cloning procedures or other laboratory manipulations, and such modifications (or variants) are also included within the scope of the present invention.

In the present invention, the flagellin may include full-length flagellin, or an active fragment thereof. In addition, terms such as "flagellin", "flagellin N-terminal constant region" and "flagellin C-terminal constant region" include naturally occurring amino acid sequences, or it may also comprise an amino acid sequence substantially identical to or similar to the amino acid sequence of a naturally occurring flagellin, flagellin N-terminal constant region, or flagellin C-terminal constant region, respectively.

In the present invention, an "active fragment" of flagellin, flagellin N-terminal constant region, flagellin C-terminal constant region, or any other portion of flagellin comprises at least about 50, 75, 100, 125, 150, 200, 250 or at least 300 contiguous amino acids and/or less than about 300, 250, 200, 150, 125, 100 or 75 amino acids of contiguous amino acids, and combinations of these may also be included as long as the lower limit is less than the upper limit. The active fragment may refer to a fragment capable of activating the TLR5 pathway in a host.

In certain embodiments, the active fragment is capable of activating the TLR5 pathway with at least about 50%, 75%, 80%, 85%, 90%, or 95% of full-length flagellin, or activates the TLR5 pathway to the same or essentially the same extent as the full-length flagellin or flagellin region, or activates the TLR5 pathway to a higher degree compared to full-length flagellin or flagellin region.

In the present invention, the active fragment may refer to at least one portion of flagellin exhibiting TLR5 pathway activity. The "at least one portion" may refer to a portion exhibiting TLR5 pathway activity in domains 0, 1, 2 and 3 of flagellin. More specifically, the active fragment may be a hypervariable region removed from full-length flagellin. The hypervariable region may vary depending on the type of bacteria from which flagellin is derived, and among the entire sequence of a specific flagellin, the sequence corresponding to the hypervariable region can be easily identified and removed by those skilled in the art. For example, N-terminal domains 0, 1, 2; domain 3; and domain 3, or domains 2 and 3 may be hypervariable regions in the case of full-length flagellin comprising C-terminal domains 2, 1, 0, and N-terminal domain 0, 1; domain 2; domain 2 may be a hypervariable region in the case of full-length flagellin including C-terminal domains 1 and 0. Alternatively, in the case of flagellin of a form not including a hypervariable region(For example, flagellin derived from many Gram-positive bacteria may not contain a hypervariable region.), the sequence of a hinge region in which folding of the flagellin protein occurs may be partially removed.

The term "hypervariable region" used in the present invention may be expressed as a propeller domain or region, a hinge, a hypervariable region, a variable domain or region, and the like.

In the present invention, the removal of the hypervariable region may mean that the entire domain corresponding to the hypervariable region may be removed, or a part of the sequence of the hypervariable region may be removed.

In the present invention, the active fragment may be flagellin in a form in which the hypervariable region of wild-type flagellin is removed and an artificial sequence(i.e., the hinge or linker of the artificial sequence) is inserted into the removed hypervariable region.

In the present invention, the flagellin fragment of the present invention may refer to a fragment exhibiting TLR5 pathway activity comprising at least one selected from the group consisting of C-terminal domain 0, C-terminal domain 1, C-terminal domain 2, N-terminal domain 2, N-terminal domain 1, N-terminal domain 0 of wild type flagellin and a region exhibiting at least 80% amino acid sequence homology with each of the domains.

In certain embodiments, the active fragment of flagellin is capable of activating the TLR5 pathway by at least about 50%, 75%, 80%, 85%, 90%, or 95% of full-length flagellin, or activating the TLR5 pathway to the same or essentially the same extent as the full-length flagellin or flagellin region, or higher activation of the TLR5 pathway compared to full-length flagellin or flagellin sites.

The present invention also includes proteins having the full-length sequence of wild-type flagellin as well as amino acid sequence variants thereof. In the present invention, the variant refers to a protein having a different sequence due to deletion, insertion, non-conservative or conservative substitutions, substitution of amino acid analogs or a combination thereof of some amino acid residues of a wild-type flagellin or a fragment thereof. Amino acid exchanges that do not entirely alter the activity of the molecule (ie, the ability to activate the TLR5 pathway) are known in the art(H. Neurath, R. L. Hill, The Proteins, Academic Press, New York, 1979).

In some cases, the variant of the present invention may be a full-length flagellin modified by phosphorylation, sulfation, acrylation, glycosylation, methylation, farnesylation or the like or a fragment thereof.

In certain embodiments, the variant of flagellin or fragment thereof is capable of activating the TLR5 pathway by at least about 50%, 75%, 80%, 85%, 90%, or 95% of full length flagellin or fragment thereof, or activating the TLR5 pathway to the same or essentially the same extent as full-length flagellin or a fragment thereof, or higher activation of the TLR5 pathway compared to full-length flagellin or a fragment thereof.

In the present invention, the flagellin, a fragment thereof, or a variant thereof may be in the form of a fusion protein comprising another polypeptide. For example, the flagellin may be a fusion protein comprising one or more antigens. Non-limiting examples of the antigen include S. *pneumoniae* PspA1 antigen, S. *pneumoniae* PspA2 antigen, S. *pneumoniae* PspA3 antigen, S. *pneumoniae* PspA4 antigen, S. *pneumoniae* PspA5 antigen and/or S. *pneumoniae* PspA6 antigen. Alternatively, for example, the flagellin may be in the form of a fusion protein to which one or more immunomodulatory substances are bound. The immunomodulatory substance may be included without limitation as long as it is known in the art to increase an immune response, and non-limiting examples thereof include interferon-α, interferon-β, interferon-γ, interferon-ω, interferon-τ, interleukin-1α, interleukin-1β, interleukin-2, interleukin-3, interleukin-4, interleukin-5, interleukin-6, interleukin-7, interleukin -8, interleukin-9, interleukin-10, interleukin-11, interleukin-12, interleukin-13, interleukin-14, interleukin-18, B cell growth factor, CD40 ligand, TNF-α, TNF-β, CCL25, CCL28 or an active fragment thereof.

As used herein, the term "percent (%) sequence homology" is defined as the percentage of amino acid residues in a candidate sequence that are identical with the amino acid residue in the reference polypeptide, after aligning the sequences and introducing gaps, without considering any conservative substitutions as part of the sequence identity to achieve the maximum percent sequence identity if necessary. Alignment for purposes of determining percent amino acid homology can be achieved, for example, using publicly available computer software programs and various methods that are within the skill in the art and using BLAST, blast-2, ALIGN, or Megalign (DNASTAR) software. One of skill in the art can determine appropriate parameters for alignment measurements, including any algorithms needed to achieve maximal alignment over the entire length of the sequences being compared. For the purposes herein, the percent (%) amino acid sequence homology of a given amino acid sequence B or of the given aminoacid sequence A to the given amino acid sequence B is calculated as follows: 100 times the fraction X/Y, where X is the number of amino acid residue scores that are identically matched by the sequence alignment program in the program alignment of A and B, and Y is the total number of the amino acid residues in B. It will be understood that if the length of amino acid sequences A is not equal to the length of amino acid sequence B, then the percent (%) amino acid sequence homology of A to B will not equal the percent (%) amino acids) sequence identity of B to A.

In certain embodiments, the flagellin, the fragment thereof, or the variant thereof may consist of an amino acid sequence selected from the group consisting of SEQ ID NOs: 1-5 or an amino acid sequence having at least 80% sequence homology thereto.

As used herein, the term "Fc region" refers to the C-terminal portion of an immunoglobulin chain constant region, and specifically refers to an immunoglobulin heavy chain constant region or a portion thereof.

The immunoglobulin Fc region of the present invention includes a native amino acid sequence as well as a mutant thereof. An amino acid sequence mutant means that one or more amino acid residues in a natural amino acid sequence have a different sequence by deletion, insertion, non-conservative or conservative substitution, or a combination thereof. For example, in the case of IgG Fc, amino acid residues 214 to 238, 297 to 299, 318 to 322, or 327 to 331 known to be important for binding may be used as suitable sites for modification. In addition, various types of mutants are possible, such as those in which a site capable of forming a disulfide bond is removed, some amino acids at the N-terminus of the native Fc are removed, or a methionine residue may be added to the N-terminus of the native Fc. In addition, in order to eliminate effector function, the complement binding site, e.g., the C1q binding site, may be removed, or the ADCC site may be removed. Techniques for preparing such an immunoglobulin Fc region sequence mutant are disclosed in International Patent Publication No. 97/34631 and International Patent Publication No. 96/32478 and the like.

Amino acid exchanges in proteins and peptides that do not entirely alter the activity of the molecule are known in the art. The most commonly occurring exchange is the exchange between the amino acid residues Ala/Ser, Val/Ile, Asp/Glu, Thr/Ser; Ala/Gly, Ala/Thr, Ser/Asn, Ala (Val), Ser/Gly (Thr/Phe), Ala/Pro, Lys/Arg (Asp/Asn), Leu/Iles, Leu/Val (Ala/Glu), Asp/Gly.

In some cases, it may be modified by phosphorylation, sulfation, acrylation, glycosylation, methylation, farnesylation, acetylation, amidation and the like.

The above-described Fc mutant is a mutant which exhibits the same biological activity as the Fc region of the present invention but has increased structural stability to heat, pH and the like of the Fc region.

In addition, such an Fc region may be obtained from natural forms isolated in vivo in animals such as humans and cattle, goats, pigs, mice, rabbits, hamsters, rats, guinea pigs and the like, or may be recombinant forms obtained from transformed animal cells or microorganisms or mutants thereof. Here, the method of obtaining from natural forms can be obtained by separating whole immunoglobulins from the body of a human or animal and then treating with protease. When treated with papain, it is cleaved into Fab and Fc, and when treated with pepsin, it is cleaved into pF'c and F(ab)2. In this case, Fc or pF'c may be separated using size-exclusion chromatography or the like.

In addition, the immunoglobulin Fc region may be a form in which a natural sugar chain, a sugar chain increased as compared with the natural form, a sugar chain decreased as compared with the natural form, or a sugar chain has been removed. Conventional methods such as chemical methods, enzymatic methods, and genetic engineering methods using microorganisms can be used to increase or eliminate the immunoglobulin Fc sugar chain. Here, the immunoglobulin Fc region from which the sugar chain has been removed from the Fc significantly reduces the binding ability of complement (c1q), reduces or eliminates antibody-dependent cytotoxicity or complement-dependent cellular cytotoxicity, and thus does not induce an unnecessary immune response in vivo. In this regard, a form more suitable for the original purpose of the drug carrier will be referred to as an immunoglobulin Fc region in which the sugar chain is removed or non-glycosylated.

In the present invention, the term "Deglycosylation" refers to an Fc region from which sugars have been removed by an enzyme, and "Aglycosylation" means a Fc region produced in prokaryotic animals, preferably Escherichia coli, and not glycosylated.

In addition, the immunoglobulin Fc region may be an Fc region derived from IgG, IgA, IgD, IgE, IgM or a combination thereof or a hybrid thereof. It may preferably be from IgG or IgM which is most abundant in human blood and most preferably from IgG which is known to enhance the half-life of the ligand binding protein.

On the other hand, in the present invention, "combination" means that when forming dimers or multimers, a polypeptide encoding a single chain immunoglobulin Fc region of the same origin forms a bond with a single chain polypeptide of different origin. That is, it is possible to prepare dimers or multimers from two or more fragments selected from the group consisting of Fc fragments of IgG Fc, IgA Fc, IgM Fc, IgD Fc and IgE.

"Hybrid" in the present invention is the term meaning that there is a sequence corresponding to an immunoglobulin Fc fragment of at least two different origins in a single immunoglobulin Fc region. Several types of hybrids are possible for the present invention. That is, a hybrid of domains of one to four domains from the group consisting of CH1, CH2, CH3 and CH4 of IgG Fc, IgM Fc, IgA Fc, IgE Fc and IgD Fc is possible and may comprise a hinge.

On the other hand, IgG can also be divided into subclasses of IgG1, IgG2, IgG3 and IgG4, and in the present invention, a combination thereof or hybridization thereof is also possible. It may preferably be derived from the Fc of human immunoglobulin IgG1.

In another embodiment, the immunoglobulin Fc region may comprise one or more selected from the group consisting of CH1, CH2, CH3 and CH4 domains of an immunoglobulin heavy chain constant region. For example, an immunoglobulin Fc region that may be used in the preparation of the fusion proteins of the present invention may comprise: (a) CH1 domain, CH2 domain, CH3 domain, and CH4 domain; (b) CH1, CH2, and CH3 domain; (c) CH1 domain, CH2 domain, and CH4 domain, (d) CH1 domain, CH3 domain, and CH4 domain; (e) CH2 domains, CH3 domain and CH4 domain: (f) CH1 domain and CH4 domain); (g) CH1 domain and CH3-domain; (h) CH1 domain and CH2 domain; (i) CH2 domain and CH4 domain; (j) CH2 domain and CH3 domain; (k) CH3 domain and CH4 domain; or a combination of two or more domains and an immunoglobulin hinge region. Most preferably, the immunoglobulin Fc region may consist of the CH2 and CH3 domain of a heavy chain constant region.

In addition to the human immunoglobulin Fc region, the present invention can also use other immunoglobulin Fc regions or fragment sequences thereof, including amino acid sequences encoded by nucleotide sequences disclosed in the GenBank and/or EMBL database, such as, for example, AF045536.1 (Macaca fascicularis), AF045537.1 (Macca mulatta), AB016710 (Felix catus), K00752 (Oryctolagus cuniculus), U03780 (Sus scra), Z48947 (Camelus Fromedarius), X62916 (Bos taurus), L07789 (Mustela vision), X6997 (Ovis aries), U17166 (Cricetulus migratorius), X07189 (Rattus ratus), AF57619.1 (Trichosurus vulpecula) or AF035195 (Monodelphis Domestica) and the like.

In the present invention, the fusion protein may be one in which the N-terminus or C-terminus of the flagellin, the fragment thereof, or the variant thereof is bound to the N-terminus or C-terminus of the immunoglobulin Fc region. Specifically, the N-terminus of the flagellin, the fragment thereof, or the variant thereof may be bound to the C-terminal of an immunoglobulin Fc region, or the C-terminus of the flagellin, the fragment thereof or the variant thereof is bound to an N-terminus of an immunoglobulins Fc region. Preferably, the C-terminus of the flagellin, the fragment thereof or the variant thereof may be bound to the N-terminal of the immunoglobulin Fc region.

In a particular embodiment, the immunoglobulin Fc region of the invention may consist of the amino acid sequence of SEQ ID NO: 6 or 7 or an amino acid sequence exhibiting at least 80% sequence homology thereto.

Meanwhile, in the present invention, each of the components constituting the fusion protein, i.e., the flagellin, the fragment thereof or the variant thereof, and the immunoglobulin Fc region may be directly connected to each other or connected via a linker. In general, the term "linker" refers to a nucleic acid, amino acid or non-peptide residue that can be inserted between one or more molecules, e.g., at least one component domain. For example, linkers can be used to provide the desired sites of interest between the components to facilitate manipulation. A linker may also be provided to enhance expression of the fusion protein from the transformant, and to reduce steric hindrance so that the component can assume its optimal tertiary structure and/or interact properly with the target molecule. The linker sequence may comprise one or more amino acids naturally linked to the receptor component, or may be an added sequence used to enhance expression of the fusion protein, to provide a desired site of specific interest, to allow the component domain to form an optimal tertiary structure, and/or to enhance interaction of the component with its target molecule.

Preferably, the linker can increase the flexibility of the fusion protein without interfering with the structure of each component in the fusion proteins. In some embodiments, the linker residue is a peptide linker having a length of 2 to 100 amino acids. Exemplary linkers include linear peptides having at least two amino acid residues, such as Gly-Gly, Gly-Ala-Gly; Gly-Pro-Ala; Gly (G)n; and Gly-Ser (GS) linkers. GS linkers described herein include, but are not limited to, (GS)n, (GSGSG)n, (G2S)n, G2S2G, (G2SG)n, (G3S)n, (G4S)n, (GGSGG)nGn, GSG4SG4SG and (GGGGS)n, where n is 1 or greater. One example of a (G)n linker includes a G9 linker, and examples of (GGGS)n linker include a GGGS or (GGGGS)3 linker. Suitable linear peptides include polyglycine, polyserine, polyproline, polyalanine and oligopeptides consisting of alanyl and/or serinyl and/ or prolinyl and/ least glycyl amino acid residues. The linker residues may be used to link the components of the fusion protein disclosed herein.

In the present invention, the linker may consist of the amino acid sequence of SEQ ID NO: 8 or 9.

The fusion proteins described herein may or may not comprise a signal peptide that functions to secrete the fusion protein from a host cell. The nucleic acid sequence encoding the signal peptide may be operably linked to the nucleic acid sequences encoding the protein of interest. In some embodiments, the fusion protein comprises a signal peptide. In some embodiments, the fusion protein does not comprise a signal peptide.

The fusion proteins described herein may also comprise modified forms of protein binding peptides. For example, the fusion protein component may have post-translational modifications including, for example, glycosylation, sialylation, acetylation and phosphorylation to any protein binding peptide.

Unless stated otherwise, the fusion proteins of the invention are administered as polypeptides (or nucleic acids encoding the polypeptides) that are themselves not part of a live, killed or recombinant bacterial or viral vectorized vaccine. Further, unless otherwise specified, the fusion proteins of the invention are isolated fusion proteins, e.g., not incorporated into flagella.

"Fusion" in the context of the present invention refers to the integration of two molecules of different or identical function or structure, which may be fusion by any physical, chemical or biological method with which the peptide may bind. The fusion protein or the polypeptide constituting the fusion protein can be produced by a chemical peptide synthesis method known in the art, or a gene encoding the fusion proteins can be amplified by PCR (polymerase chain reaction) or synthesized by a known method, and then cloned into an expression vector and expressed.

In certain embodiments of the invention the fusion protein may consist of an amino acid sequence selected from the group consisting of SEQ ID NOs: 10-16.

The invention also provides a polynucleotide comprising a nucleotide sequence encoding the fusion protein.

The polynucleotide is not particularly limited in the combination of bases constituting the polynucleotide as long as it can encode the polypeptide of the present invention. The polynucleotide can be provided as a nucleic acid molecule in the form of a single strand or a double strand, including both DNA, cDNA and RNA sequences.

Preferably, the polynucleotide of the present invention may have a nucleotide sequence selected from the group consisting of SEQ ID NOs: 17 to 23.

The invention also provides a vector comprising the polynucleotide.

Vectors of the invention include, but are not limited to, plasmid vectors, cosmid vectors, bacteriophage vectors, viral vectors, and the like. The vector of the present invention may be a conventional cloning vector or an expression vector, and the expression vector may comprise a signal sequence or a leader sequence for membrane targeting or secretion in addition to expression control sequences such as a promoter, an operator, an initiation codon, a termination codon, polyadenylation signal and an enhancer (a promoter), and may be variously prepared according to the purpose. The polynucleotide sequence according to the present invention may be operably linked to an expression control sequence, and the operably linked gene sequence and the expression control sequences may be comprised in one expression vector containing a selection marker and a replication origin. "Operably linked" refers to the linkage of an appropriate molecule to an expression control sequence in a manner that enables gene expression, wherein one nucleic acid fragment is linked to another nucleic acid fragment such that its function or expression is affected by the other. "Expression control sequence" means a DNA sequence that regulates the expression of a polynucleotide sequence operably linked in a particular host cell. Such regulatory sequences include a promoter to effect transcription, any operator sequence to control transcription, a sequence encoding a suitable mRNA ribosome binding site, and a sequence that controls the termination of transcription and translation. The vector also includes a selection marker for selecting a host cell containing the vector, and includes a origin of replication if it is a replicable vector.

The invention also provides a transformant transformed with the vector.

Transformation with the vectors can be carried out by transformation techniques known to those skilled in the art. Preferably microprojectile bombardment, electroporation, calcium phosphate (CaPO₄) precipitation, calcium chloride (CaCl₂) precipitation, PEG-mediated fusion, microinjection and liposome-mediated methods can be used.

The term "transformant" may be used interchangeably with "host cell" and refers to a prokaryotic or eukaryotic cell comprising heterologous DNA introduced into the cell by any means (e.g., electroporation, calcium phosphatase precipitation, microinjection, transformation, viral infection, etc.).

In the present invention, the transformant can be used as a host cell for all kinds of single cell organisms commonly used in the cloning field, for example, prokaryotic cells such as various bacteria (e.g., genus *Clostridia, E. coli,* etc.), lower eukaryotic cells such as yeast and cells derived from higher eukaryotic organisms including insect cells, plant cells, mammals, and the like, but not limited thereto. Since the expression level and modification of the protein appear differently depending on the host cell, a person skilled in the art can select and use the most suitable host cell for the intended purpose.

The present invention also provides a pharmaceutical composition comprising the fusion protein as an active ingredient.

According to one embodiment of the present invention, it has been found that the fusion protein exhibits significantly improved TLR5 pathway activating ability compared to wild type flagellin. Thus, the fusion protein of the present invention may exhibit preventive, ameliorated or therapeutic effects on diseases, syndromes, etc. known to be preventable, ameliorated or treatable through activation of the TLR5 pathway.

Diseases, syndromes known to be preventable, ameliorated or treatable through activation of the TLR5 pathway may be damage by radiation exposure; reperfusion injury; inflammatory bowel disease; autoimmune disease; viral infection; aging; immune function decline; or cancer.

Thus, the pharmaceutical composition of the present invention can be characterized as a pharmaceutical composition for preventing or treating damage caused by radiation exposure; for prevention or treating reperfusion injury; for the prevention or treatment of inflammatory bowel disease; for the prevention or treatment of autoimmune diseases; for preventing or treating viral infections; for prevention or treating aging; for enhancing immune function; or for the treatment of cancer.

In particular, the fusion protein of the present invention may be understood to exert a preventive, ameliorative or therapeutic effect on diseases which will be found in the future to be able to be prevented, ameliorated or treated through TLR5 pathway activation, and thus the disease to be treated of the pharmaceutical compositions of the invention is not particularly limited in scope.

The association of the activation of the TLR5 pathway with the treatment of damage by radiation exposure may refer to KR20067010934A and the like, the association of activation of TLR5 pathways with treatment of tissue damage due to reperfusion may refer to US8324163 and the like, the association of activating TLR5 pathway with treatment for inflammatory bowel disease may refer to US7361733 and the like, the association activating TLR 5 pathway with the therapy for autoimmune diseases may refer to EP03010523B1 and the like, the associated with activation TLR 5 pathway with therapy of viral infections may refer to US9872895 and the like, the relationship between activation of the TLR5 pathway and diseases caused by aging may refer to KR20150049811A and the like, the relationship between the activation of the TLR5 pathway and the enhancement of immunity may refer to WO17031280A1 and the like, the relationship between activation of the TLR5 pathway and cancer treatment may refer to KR20177005615A and the like.

In the present invention, the damage by radiation exposure may be a gastrointestinal syndrome or a hematopoietic syndrome due to radiation exposure.

In the present invention, the disease caused by aging may be hair loss due to aging, cataract, hair loss, colitis, osteoporosis or osteomalacia.

In the present invention, the cancer may be breast cancer, lung cancer, colon cancer, kidney cancer, liver cancer, ovarian cancer, prostate cancer, testicular cancer, genitourinary duct cancer, lymphatic system cancer, rectal cancer, pancreatic cancer, esophageal cancer, stomach cancer, cervical cancer, thyroid cancer, skin cancer, leukemia, acute lymphocytic leukemias, acute lymphoblastic leukemia, B-cell lymphoma, T cell lymphoma, Hodgkin lymphoma, non-Hodgkin lymphoma, hairy cell lymphomas, histiocytic lymphoma and Burkitt lymphoma, acute and chronic myelogenous leukemia, myelodysplastic syndrome, myeloid leukemia, promyelocytic leukemia, astrocytoma, neuroblastoma, glioma, schwannoma, fibrosarcoma, rhabdomyosarcoma, osteosarcoma, xeroderma pigmentosum, keratoctanthoma, seminoma, thyroid follicular cancer, teratocarcinoma, or cancer of the gastrointestinal tract.

The pharmaceutical compositions of the present invention may be formulated with a pharmaceutically acceptable carrier in addition to the fusion protein in a manner known in the art according to the route of administration. "Pharmaceutically acceptable" refers to a non-toxic material that is physiologically acceptable and that does not interfere with the action of the active ingredient and does not normally produce an allergic or similar reaction, such as gastrointestinal disorders, dizziness, when administered to a human. Such carriers include all kinds of solvents, dispersion media, oil-in-water or water-oil emulsions, aqueous compositions, liposomes, microbeads and microsomes.

Routes of administration may be orally or parenterally. Parenteral methods of administration include, but are not limited to, intravenous, intramuscular, intraarterial, intramedullary, intrathecal, intracardiac, transdermal, subcutaneous, intraperitoneal, intranasal, enteral, topical, sublingual, or rectal administration.

In the case of oral administration of the pharmaceutical composition of the present invention, it can be formulated into the form of powders, granules, tablets, pills, dragees, capsules, liquids, gels, syrups, suspensions, wafers and the like according to methods known in the art together with a suitable oral carrier. Examples of suitable carriers may include sugars, including lactose, dextrose, sucrose, sorbitol, mannitol, xylitol, erythritol and maltitol and the like, and starches, including corn starch, wheat starch, rice starch and potato starch and the like, celluloses, including cellulose, methyl cellulose, sodium carboxymethylcellulose and hydroxypropylmethylcellulose and the like, and fillers, such as gelatin, polyvinylpyrrolidone, and the like. Also, if desired, cross-linked polyvinylpyrrolidone, agar, alginic acid or sodium alginate and the like may be added as a disintegrant. Furthermore, the pharmaceutical composition may further comprise anti-agglomerants, lubricants, wetting agents, flavoring agents, emulsifying agents, preservatives and the like.

In addition, for parenteral administration, the pharmaceutical composition of the present invention can be formulated in the form of an injection, a transdermal administration and a nasal inhalation, together with a suitable parenteral carrier, according to a method known in the art. In the case of the injection, it must be sterilized and protected from contamination of microorganisms such as bacteria and fungi. Examples of suitable carriers for injection can be solvents or dispersion media including, but not limited to, water, ethanol, polyols (such as glycerol, propylene glycol, and liquid polyethylene glycol and the like), mixtures thereof, and/or vegetable oils. More preferably, as suitable carriers, Hanks' solution, Ringer's solution, phosphate buffered saline (PBS) or isotonic solutions such as sterile water for injection, 10% ethanol, 40% propylene glycol and 5% dextrose, and the like can be used. To protect the injection from microbial contamination, various antibacterial and antifungal agents, such as paraben, chlorobutanol, phenol, sorbic acid, thimerosal and the like, can be further included. In addition, the injection may in most cases further comprise isotonic agents such as sugars or sodium chloride.

In the case of transdermal administration, forms such as ointments, creams, lotions, gels, external solutions, pasta preparations, liniments, aerosols and the like are included. In the above, "transdermal administration" means that a pharmaceutical composition is topically administered to the skin so that an effective amount of the active ingredient contained in the pharmaceutical composition is delivered into the skin. For example, the pharmaceutical composition of the present invention may be administered by a method in which it is prepared in an injectable formulation and the skin is pricked or applied directly to the skin with a 30-gauge fine injection needle. These formulations are described in a formulation generally known in pharmaceutical chemistry.

In the case of inhalation administration, the compounds used in accordance with the invention can be conveniently delivered in the form of an aerosol spray from pressurized packs or a nebulizer, with the use of a suitable propellant, e.g., dichlorofluoromethane, trichlorofluoromethane, dichlorotetrafluoroethane, carbon dioxide or other suitable gas. In the case of a pressurized aerosol, the dosage unit may be determined by providing a valve to deliver a metered amount. For example, gelatin capsules and cartridges for use in an inhaler or insufflator may be formulated to contain a powder mix of the compound and a suitable powder base such as lactose or starch.

As other pharmaceutically acceptable carriers, those known in the art can be referred to.

In addition, the pharmaceutical compositions according to the present invention may further comprise one or more buffers (such as saline or PBS), carbohydrates (such as glucose, mannose, sucrose, or dextran), antioxidants, bacteriostats, chelating agents (such as EDTA or glutathione), adjuvants (such as aluminum hydroxide), suspending agents, thickening agents, and/or preservatives.

The pharmaceutical compositions of the invention may also be formulated using methods known in the art to provide quick, sustained or delayed release of the active ingredient after administration to a mammal.

In addition, the pharmaceutical composition of the present invention can be administered in combination with known substances having the effect of preventing or treating the respective diseases listed above.

The present invention also provides a vaccine adjuvant comprising the fusion protein as an active ingredient.

One of the most important requirements of vaccine adjuvants is to have immunomodulatory functions such as regulation of costimulatory molecule expression on the surface of antigen presenting cells and regulation of cytokine secretion due to induction of antigen-specific T cells.

By the way, PRRs such as TLR5 are distributed in the cell surface or cytoplasm of host cells, and induce 'innate immune response' by stimulation of various PAMPs, and further regulate 'adaptive immune response.' Thus, TLR5 agonists may be suitable targets for the development of various 'immunomodulators', particularly 'vaccine adjuvants'.

Thus, the fusion proteins of the present invention that have the ability to activate the TLR5 pathway can activate TLR5 pathways to enhance innate immune responses and acquired immune responses, so that the host's immunity to the co-administered antigen can be significantly improved.

The vaccine adjuvants of the present invention may be prepared by conventional methods well known in the art, and may optionally further comprise various additives that may be used in preparing vaccines in the art.

The present invention also provides a use of the fusion protein for the preparation of a pharmaceutical agent for the treatment of injury by radiation exposure; the treatment of reperfusion injury; the treatment of inflammatory bowel disease; the treatment of autoimmune disease; the treatment of viral infection; the treatment of metabolic disease; the treatment of aging; the enhancement of immune function; or the treatment of cancer.

In addition, the present invention provides a method for treating injury by exposure to radiation; treating reperfusion injury; treating inflammatory bowel disease; treating autoimmune disease, treating viral infection; treating metabolic disease; treating aging; enhancing immune function; or treating cancer, comprising administering to a subject in need thereof an effective amount of a composition comprising the fusion protein as an active ingredient.

The 'effective amount' of the present invention refers to an amount that, when administered to a subject, exhibits the effect of improving, treating, preventing, detecting, diagnosing, or inhibiting or reducing the damage caused by radiation exposure; reperfusion injury; inflammatory bowel disease; autoimmune diseases; viral infection; metabolic diseases; aging; boosting immune function; or cancer. The 'subject' may be an animal, preferably a mammal, in particular an animal including a human, and animal derived cells, tissues, organs, etc. The subject may be a patient in need of the effect.

The 'treatment' of the present invention refers broadly to ameliorating injury by radiation exposure; reperfusion injury; inflammatory bowel disease; autoimmune disease; viral infection; metabolic disease; aging; immune function enhancement; or cancer, or symptoms of the disease, which may include, but are not limited to, curing, substantially preventing, or ameliorating the condition of, and which includes ameliorating, curing or preventing one or most symptoms resulting from the disease.

As used herein, the term 'comprising' is used in the same sense as 'including' or 'characterized by', and in the composition or method according to the present invention, additional components or steps of the method not specifically mentioned are not excluded. In addition, the term 'consisting of' means excluding additional elements, steps, or ingredients that are not separately described. The term 'essentially consisting of' means that, in the scope of the composition or method, it may include substances or steps that do not substantially affect the basic properties thereof in addition to the substances or steps described.

### ADVANTAGEOUS EFFECT

The fusion protein provided by the present invention has significantly superior toll-like receptor 5 (TLR5) pathway activation ability compared to a wild-type flagellin, a fragment thereof or a variant thereof, and it can be very usefully utilized in the development of therapeutic agents and/or vaccine adjuvants for diseases that can be prevented, improved, or treated through activation of the TLR5 pathway.

### BRIEF DESCRIPTION OF THE DRAWINGS

FIG. 1 is a schematic diagram simply showing the preparation of Fc fusion flagellin of various microorganisms-derived flagellin and human IgG4-Fc (hIgG4) provided in the present invention.
FIG. 2 is the result of confirming cloning by culturing colonies of each strain cutting with enzymes, and checking the size of flagellin and vector of each strain to confirm the fusion of the prepared microorganism-derived Fc-hIgG4-flagellin(BS, BsFlagellin; EC, EcFlagellin; PA, PaFlagellin; SD, SdFlagellin; SH, ShFlagellin).
FIG. 3 is a result showing that each Fc-hIgG4-flagellin fusion protein forms a dimer.
FIG. 4 is a result showing the comparison of TLR5-dependent NF-κB activity by Fc-hIgG4-flagellin fusion proteins derived from each microorganism.
FIG. 5 is a schematic diagram of the production of Fc-mIgG1-Bsflagellin prepared for animal experiments of Bsflagelllin, which induces the highest TLR5 activity. A fusion protein was constructed using two linkers of different lengths.
FIG. 6 is a result confirming the cloning of Fc-mIgG1-Bsflagellin prepared using two linkers of different lengths.
FIG. 7 is a result of confirming the expression of the fusion protein of the present invention through Western blot(#1: Fc-mIgG1-linker-bsflagellin, #2: Fc-mIgG1-linker*3-bsflagellin).
FIG. 8 is a result of confirming through Western blot whether the fusion protein of the present invention forms a dimer.
FIG. 9 is a result of evaluating (FLA-ST) NF-κB activation ability of an Fc-fused bsflagellin (mIgG1-Fc2-Bsflagellin) and purified flagellin protein (FLA-BS) of *Bacillus subtilis,* and purified flagellin protein of *Salmonella typhimurim.*

### MODE FOR CARRYING OUT INVENTION

Hereinafter, the present invention will be described in detail.

However, the following examples are only illustrative of the present invention, and the content of the present invention is not limited to the following examples.

### Example 1: Preparation of Fc-flagellin fusion protein (cloning)

### (1) Experimental method

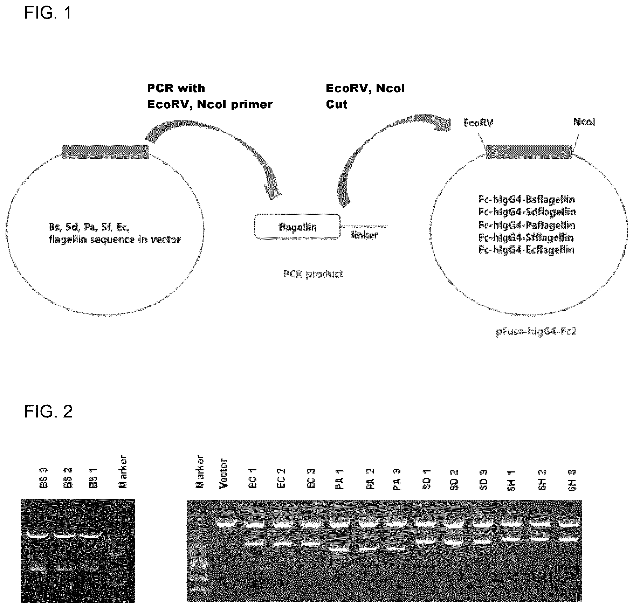

It was attempted to produce an Fc-flagellin fusion protein in which flagellin derived from various microorganisms and the Fc region of an antibody were fused (FIG. 1).

As shown in FIG. 1, cloning was performed according to the following method in order to construct plasmids each expressing a form in which flagellin derived from *Bacillus subtilis* (Bs), *Salmonella dublin* (Sd), *Pseudomonas aeruginosa* (Pa), *Shigella flexneri* (Sf) and *Escherichia coli* (Ec) was bound to human IgG4-Fc (hIgG4).
1) PCR was performed for all flagellins using EcoRV and NcoI restriction enzyme site primers (Table 2) according to the conditions shown in Table 1 below:

**Table 1**

| | | **Negative Control** | **EcoRV-Bsflagelli n-NcoI** | **EcoRV-Ecflagelli n-NcoI** | **EcoRV-Sdflagelli n-NcoI** | **Eco Sffl n-N RV-agelli coI** | **EcoRV-Paflagelli n-NcoI** |
|---|---|---|---|---|---|---|---|
| 10X Buffer | | 5 uL | 5 uL | 5 uL | 5 uL | 5 uL | 5 uL |
| dNTP | | 1 uL | 1 uL | 1 uL | 1 uL | 1 uL | 1 uL |
| Forward Primer | | 2 uL | 2 uL | 2 uL | 2 uL | 2 uL | 2 uL |
| Reverse Primer | | 2 uL | 2 uL | 2 uL | 2 uL | 2 uL | 2 uL |
| Template | | 1 uL | 1 uL | 1 uL | 1 uL | 1 uL | 1 uL |
| PCR Enzyme | | 0 uL | 1 uL | 1 uL | 1 uL | 1 uL | 1 uL |
| DW | | Up to 50 uL | Up to 50 uL | Up to 50 uL | Up to 50 uL | Up to 50 uL | Up to 50 uL |
| PCR Cycle | | | | | | | |
| | 1. 95°C for 2 min | | | | | | |
| | 2. 95°C for 20 secs | | | | | | |
| | 3. 65°C for 40 secs | | | | | | |
| | 4. 72°C for 1 min à go to step 2 x29 | | | | | | |
| | 5. 72°C for 5 min | | | | | | |

**Table 2**

| **Division** | **Primer sequence** |
|---|---|
| Ecflagellin-hIgG4-Fc2-5' EcoRV-**G** | 5' CCGGATATCGAT GGC ACA AGT CAT TAA 3' TAC CAA CAG CCT CTC G( SEQ ID NO: 24) |
| Ecflagellin-(GGGGS)-hIgG1-Fc2-3' Nco I-**C** | 5' GACCATGGC (AGA CCC TCC GCC ACC) 3' CTG CAG CAG AGA CAG AAC CTG CTG( SEQ ID NO: 25) |
| Paflagellin-h IgG4-Fc2-5' EcoRV-**G** | 5' CCGGATATCGAT GGC CTT GAC CGT CAA 3' CAC CAA CAT CGC TTC G( SEQ ID NO: 26) |
| Paflagellin-(GGGGS)-hIgG1-Fc2-3' Nco I-**C** | 5' GACCATGGC (AGA CCC TCC GCC ACC) 3' GCG CAG CAG GCT CAG AAC CGA CTG CGG( SEQ ID NO: 27) |
| Sdflagellin-hIgG4-Fc2-5' EcoRV-**G** | 5' CCGGATATCGAT GGC ACA AGT CAT TAA 3' TAC AAA CAG C( SEQ ID NO: 28) |
| Sdflagellin-(GGGGS)-hIgG1-Fc2-3' Nco I-**C** | 5' GACCATGGC (AGA CCC TCC GCC ACC) 3' ACG CAG TAA AGA GAG GAC GTT TTG( SEQ ID NO: 29) |
| Sfflagellin-hIgG4-Fc2-5' EcoRV-**G** | 5' CCGGATATCGAT GGC ACA AGT CAT TAA 3' TAC CAA CAG C(SEQ ID NO: 30) |
| Sfflagellin-(GGGGS)-hIgG1-Fc2-3' Nco I-**C** | 5’ GACCATGGC (AGA CCC TCC GCC ACC) 3’ ACC CTG CAG CAG AGA CAG AAC CTG( SEQ ID NO: 31) |
| Bsflagellin-hIgG4-Fc2-5' EcoRV-**G** | 5' C CGGATATCG ATG AGA ATT AAC CAC 3' AAT ATT GCA GCA CTT AAC( SEQ ID NO: 32) |
| Bsflagellin-(GGGGS)-hIgG1-Fc2-3' Nco I-**C** | 5’ GACCATGGC (AGA CCC TCC GCC ACC) 3’ ACG TAA TAA TTG AAG TAC GTT TTG AGG CTG( SEQ ID NO: 33) |
| √ Bold type G and C are the nucleotide sequences inserted to match the Open Reading Frame | |

2) After adding 4.4 uL of 10x DNA dye to the PCR product, gel electrophoresis was performed, the size was checked, and the flagellin band was extracted using MEGAquick-spinTM plus (cat#17290).
3) Purified DNA was digested with EcoRV and NCol according to the conditions in Table 3 below:

**Table 3**

| | **pFUSE-hlgG4-Fc2** | **EcoRV-Bsflagelli n-NcoI** | **EcoRV-Esflagelli n-NcoI** | **EcoRV- Sdflagelli n-NcoI** | **EcoRV-Sfflagelli n-NcoI** | **EcoRV-Paflagelli n-NcoI** |
|---|---|---|---|---|---|---|
| 10X K | 4 uL | 4 uL | 4 uL | 4 uL | 4 uL | 4 uL |
| DNA | 1 ug | 1 ug | 1 ug | 1 ug | 1 ug | 1 ug |
| EcoRV | 1 uL | 1 uL | 1 uL | 1 uL | 1 uL | 1 uL |
| NcoI | 1 uL | 1 uL | 1 uL | 1 uL | 1 uL | 1 uL |
| BSA 0.1 % | 2 uL | 2 uL | 2 uL | 2 uL | 2 uL | 2 uL |
| DW | Up to 40 uL | Up to 40 uL | Up to 40 uL | Up to 40 uL | Up to 40 uL | Up to 40 uL |
| | For 20hrs at 37°C | | | | | |

4) 1ul of Caff Alkaline Phosphatase was added to the vector cut sample and reacted at 37°C for 2 hours.
5) After gel electrophoresis by adding 4.4 uL of 10x DNA dye, each band was extracted using MEGAquick-spinTM plus (cat#17290).
6) Molecular ratio Vector: Insert = 1:3, and ligation was performed according to the conditions in Table 4 below.

**Table 4**

| | | **V+Bs** | **V+Ec** | **V+Sd** | **V+Sf** | **V+Pa** | **Nagative control** |
|---|---|---|---|---|---|---|---|
| 10X B | | 1 uL | 1 uL | 1 uL | 1 uL | 1 uL | 1 uL |
| Vector | | 1 uL | 1 uL | 1 uL | 1 uL | 1 uL | 1 uL |
| Ligase | | 0.5 uL | 0.5 uL | 0.5 uL | 0.5 uL | 0.5 uL | 0.5 uL |
| Insert | | 1.5 uL | 3.15 uL | 3.51 uL | 2.92 uL | 3.80 uL | 0 uL |
| DW | | Up to 10 uL | Up to 10 uL | Up to 10 uL | Up to 10 uL | Up to 10 uL | Up to 10 uL |
| | For 16hrs at 16°C | | | | | | |
| | * Vector Conc. : 4.5/4.1kb | | | | | | |
| | Bs: 2.5/845 bp | | | | | | |
| | Ec: 1.5/1.5kb | | | | | | |
| | Sd: 2.5/1.5 kb | | | | | | |
| | Sf: 2.4/1.6 kb | | | | | | |
| | Pa: 2.5/1.2 kb | | | | | | |

7) Transformation was carried out in Zeocin(+) LB plate and cultured for 18 hours.
8) Mini Prep was performed and enzymatic digestion was performed using EcoRV and NcoI.

### (2) Experiment results

First, as a result of gel electrophoresis by picking three colonies each, as shown in FIG. 2, all five types of Bsflagellin, Ecflagellin, Paflagellin, Sdflagellin, and Shflagellin were confirmed to have the same vector size, and it was confirmed that the sequence was 100% identical without mutation through sequencing.

### Example 2: Preparation of Fc-hIgG4-flagellin fusion protein (separation and purification)

### (1) Experimental method

- 5 mL of Protein A Resin [Repligen, #10-2500-01] is washed three times in total with 20 mL of Binding Buffer (pH 5.0; contains 0.02% sodium azide) [Thermo Fisher Scientific, 21019].
1) Resin is allowed to naturally settle for 5 minutes each time upon washing, and then the supernatant is removed.
   - The washed Resin is slowly poured into Disposable Columns [Thermo Fisher Scientific, 29920]. The Column and Resin are brought to the same state by pouring a Binding Buffer into the prepared Columm.
   - The cell culture prepared at room temperature is then slowly poured into the Column. The solution is then slowly flowed out of the Column. Once all of the cell culture is removed, the Column is washed with Binding Buffer.
   - After the neutralization solution (1M Tris, pH 9.2) was pre-soaked in a microfuge tube prior to elution, Column was put into the prepared microfuge tube and Elution Buffer (pH 2.8; amine-based) [Thermo Fisher Scientific, 21004] was put in good mixing with Resin. After waiting until all of the Resin sinks, the solution is allowed to flow slowly.
   - Spin Desalting Column [Thermo Fisher Scientific, 89891] is used to change the buffer solution of the isolated protein to Phosphate Buffered Saline (PBS).
      1) Equilibrate 5 ml PBS in Column for a total of 5 times, then slowly pour the extracted protein solution into Column and run a portion of the initial solution. The PBS solution is again added to the Column and the solution is slowly added to a new tube.
   - Endotoxin present in the protein solution is removed using Endotoxin Removal Spin Column [Thermo Fisher Scientific, 88273].
      1) The Spin Column is placed in a tube and centrifuged at a speed of 500 g for 1 minute to evacuate the solution contained therein, followed by 2M Sodium Chloride (NaCl) and slowly shaking the Column until the Resin was suspended in the solution. Column is placed in a tube and centrifuged for 1 minute at a speed of 500 g to evacuate the solution contained therein.
      2) Adding pure water free of endotoxin to the Resin, gently shaking the Column until the solution is suspended, then placing the Column in a tube and centrifuging for 1 minute at a speed of 500 g to evacuate the solution contained therein.
      3) Thereafter, Buffer (25 mM Sodium Phosphate, pH 7.0) without endotoxin was added and Column was gently shaken until the Resin was suspended in the solution. Column is placed to the tube and centrifuged at 500 g speed for 1 minute to evacuate the solution contained therein. This process is repeated twice.
      4) The extracted protein solution is slowly poured and the Column is gently shaken until the Resin is suspended in the solution, followed by incubation at 4°C for 1 hour with gentle ramp-down.
      5) The Column is placed in a tube and centrifuged at a speed of 500 g for 1 minute to obtain the solution contained in the tube.
   - The degree of endotoxin of the purified protein solution is measured using the LAL Chromogenic Endotoxin Quantitation Kit [Thermo Fisher Scientific, 88282].
      1) To measure the standard curve in a 96-well plate at 37°C, 50 µL each of pure water without endotoxin (0 EU/mL) and standard protein solution (0.1, 0.25, 0.5, 1 EU/ml) are added to the wells.
      2) 50 µL of the purified protein solution is added to two wells per sample.
      3) 50 µL of Limulus Amebocyte Lysate (LAL) reagent is added to each well, and then the plate is gently shaken and mixed and incubated at 37°C for 10 minutes. After 10 minutes, 100 µL of the Chromogenic Substrate reagent was added to each well, and the plate was gently shaken to mix and incubated at 37°C for 6 minutes. After 6 minutes, 100 µL of Stop Reagent (25% acetic acid) was added to each well and the plate was gently mixed at room temperature.
      4) The optical density (OD) value is measured directly on a micro-multireader machine with a wavelength of 410 nm.
      5) After the standard curve is drawn with the OD value obtained in the standard protein solution, the fitting function in exponential form is identified, and then the OD values obtained from the purified protein solution are fitted to the fitting function to determine the endotoxin concentration.
   - The concentration of the final harvested purified protein solution is measured using the BCA Protein Assay Kit [Thermo Fisher Scientific, 23227].
      1) To a 96-well plate, a purified protein solution, PBS (0 ug/mL), and a standard protein solution were serially diluted in PBS to prepare standard protein solutions (25, 125, 250, 500, 750, 1000, 1500, and 2000 ug/mL) each at a concentration of 10 µL per well.
      2) Solution A and Solution B enclosed in Kit were mixed at a ratio of 50:1, 200 µL each was added to each well, and the mixture was incubated for 30 minutes in a 37°C incubator without light irradiation.
      3) The OD value is measured immediately after 30 minutes in a micro multireader with a wavelength of 562nm.
      4) The protein concentration is determined by determining the fitting function in exponential form after a standard curve is drawn with the OD value obtained in the standard protein solution and then substituting the OD value obtained in the purified protein solution into a fitting function.

### (2) Experimental Results

The concentration of each protein after separation and purification according to the above experimental method is shown in Table 5.

**Table 5**

| | **Plasmid DNA prep** | **Transfection (100 mL)** | **Quantification** | | **Endotoxin level (EU/mL)** |
|---|---|---|---|---|---|
| | | | **mg** | **mg/L** | |
| **Fc-hIgG4-Ecflagellin** | Done | Done | 0.25 | 2.5 | 12.3 |
| **Fc-hIgG4-Sdflagellin** | Done | Done | 0.41 | 4.1 | 6.8 |
| **Fc-hIgG4-Sfflagellin** | Done | Done | 0.08 | 0.8 | 0.1 |
| **Fc-hIgG4-Bsflagellin** | Done | Done | 2.15 | 21.5 | 7.4 |
| **Fc-hIgG4-Paflagellin** | Done | Done | 0.96 | 9.6 | 11.0 |

### Example 3: Confirmation of Dimerization of Fusion Proteins

An attempt was made to determine whether the Fc-flagellin fusion protein of various strains in which the expression of the protein was confirmed forms a dimer.
(1) Experimental Method
   - Cell line used: HEK293T
   - 24 well plates are seeded to a cell confluence of 70% on the day of transfection.
   - On the day of the experiment, JetPrime transfection reagent was mixed with 500 ng of each DNA (EV, #1, #2, TdTmt) in an amount of 1 ul per well, followed by transfection according to the protocol (for transfection, 10% FBS DMEM was used. Transfection volume is 500 ul).
   - 24 hours after transfection, it is switched to 0.5% FBS media.
   - harvest the media 400 ul after 48 hours.
   - Media is centrifuged at 12,300g for 1 minute to remove debris and the supernatant is used as a sample.
   - 50 ul of two kinds of 5x sample buffer are added by dividing each sample by 200 ul.
      1) Reducing 5x sample buffer: a commonly used Laemmli sample buffer composition
      2) Non-Reducing 5x sample buffer: no β-mercaptoethanol was added in the Laemmli sample buffer composition.
         - Heat reducing samples at 99°C heat block for 5 minutes.
         - Non-reducing samples omit the heating process
         - Separate by size of protein by SDS-PAGE.
         - Check the size by Commasie staining.
(2) Experimental Results

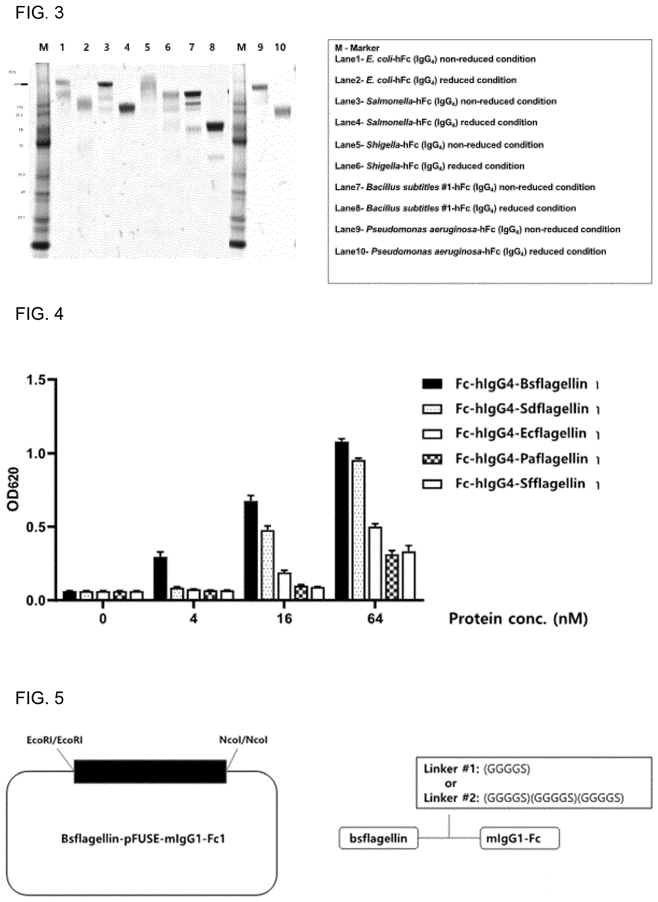

The results are shown in FIG. 3. Under Non-reducing conditions, a dimer formation signal corresponding to approximately twice the size from which the signal appeared at reducing conditions was observed for each Fc-flagellin fusion protein. This confirmed that the Fc-flagellin fusion protein forms a dimer.

### Example 4: Evaluation of TLR5 pathway activation ability of Fc-flagellin fusion protein

In order to confirm whether the prepared Fc-flagellin fusion protein exhibits activity as a TLR5 agonist, intracellular NF-κB activity was evaluated. As the cells, HEK-Blue hTLR5 (Invivogen, Cat No. hkb-htrl5), which is a cell line model constructed for selecting an agent that induces TLR5 activity to HEK293T, was used, and TLR5 activities by Fc-flagellin prepared by overexpression of hTL R5 and pNF-κB can be confirmed.

### (1) Experimental Method

HEK-Blu hTLR5 cells are maintained in culture medium containing Zeocin (100 ug/mL) (Invivogne, Cat No. ant-zn-1), blasticidin (15 ug or mL) (Invivogen, Ct No. at-bl-1), and normocin (100 u g/mL (invivoogen, C at No. anti-nr-1).
- The method for measuring NF-kB activity is as follows:
   ① The cultured HEK-Blue hTLR5 cells are detached, centrifuged, the supernatant is removed, and the cell pellet is mixed well with 1×PBS.
   ② Prior to seeding the cells, pre-prepared protein samples (20 ul) at concentrations of 4 nM, 16 nM, and 64 nM, respectively, are added to 96-well plates in advance.
   ③ The experiment was conducted under Tri-plates conditions.
   ④ The control group is a group added with 1×PBS (20 ul).
   ⑤ Calculated HEK-Blue hTLR5 cells were mixed well with HEK-BLUE Dection medium (Invivogen, Cat No. hb-det2) and seeded in 96-well plates at approximately 2.5×10⁴/96-well with equal cell numbers in 180 ul volume.
   ⑥ Cells added with protein are reacted in a 37°C incubator for about 16 hours.
   ⑦ After about 16 hours, the NF-kB activity is compared and calculated by measuring the optical density (OD) values at 620 nm wavelength in a micro multireader.

### (2) Experimental Results

The results are shown in FIG. 4.

As shown in FIG. 4, each of the Fc-hIgG4-flagellin fusion proteins was found to increase TLR5-dependent NF-κB activity in a concentration-dependent manner, and in particular, it was confirmed that the activity of the Fc-hIgG4-bsflagellin fusion protein was the most excellent at all concentrations. It was found that other Fc-hIgG4-flagellin fusion proteins at low concentrations of 4 nM do not induce the activity of TLR5, whereas only Fc-HIgG4-bs flagellin is capable of inducing significant TLR5 activity.

### Example 5: Preparation of Fc-mIgG1-bsflagellin fusion protein for animal experiments

In order to validate the Fc-hIgG4-Bsflagellin identified as showing the most excellent activity in Examples 1 to 4 above in a mouse animal model, a vector comprising a polynucleotide encoding a protein fused with an Fc region derived from mouse IgG1 was prepared as follows (FIG. 5). In order to compare whether the linker size affects the function of Fc fusion flagelllin, one linker GGGGS (SEQ ID NO: 8) and three linkers GGGS (Seq. ID NO: 9) were used, respectively.

A vector comprising a polynucleotide encoding a protein fused with a Bsflagelliin, a linker and an Fc region derived from mouse IgG1 was prepared as follows (FIG. 5).

Specifically, PCR was performed in a pET49b-bsflagellin vector (Scientific Reports, 7, 40878 (2017)) in which the *B*. *subtilis* flagellin (bs flagelin) gene was inserted using the forward primer (5'-CCG GAATTCATGAGAATTAACCACAATATTGCAGCACTTAAC-3) (SEQ ID NO: 34) and the reverse primers (5'-GACCATGGTAGACCCTCCGCCACCACGTAATAATTGAAGTACGTTTTTGAGGCTG-3' (SEQ NO: 35) and 5'-GACCATGGTAGACCCTCCACCACCACGACCTCCGCCACCACGTAATAATTGAGTAC GTTTGAGGCTG-3'-(SEQ ID No: 36)). PCR is performed using pfu turbo DNA polymerase (Invitrogen, 2.5 unit/ul, #600252) at 92°C for 30 seconds, at 92°C for 30 seconds, at 56°C for 30 seconds, at 68°C for 1 minute as 1 cycle. After 30 cycles, the reaction was terminated by reacting at 68°C for 5 minutes.

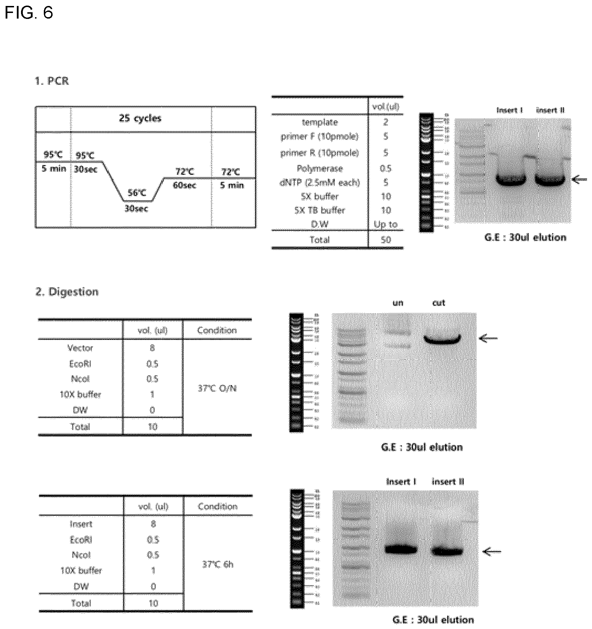

The PCR product thus obtained was treated with EcoRI/NcoI and then ligated into the pFUSE-mIgG1-Fc1 vector treated with the EcoR1/Nco I enzyme using T4 DNA ligase (Takara, #2011A) to confirm the expression vector of "pFUSE-mIgG1-Fc1-bs flagellin" by sequencing (FIG. 6).

The expression of the Fc-Bsflagellin fusion protein was confirmed after transfection of cells with the vector prepared above.

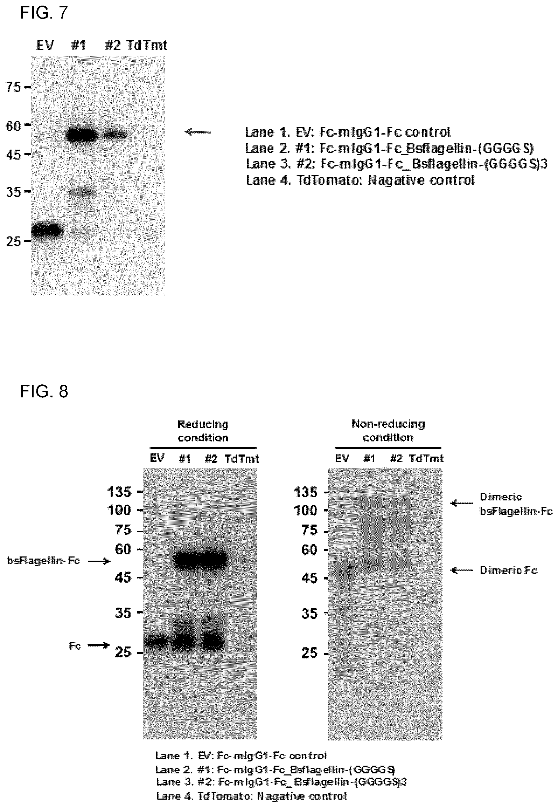

The results are shown in FIG. 7.

As can be seen from FIG. 7, unconjugated Fc protein (26 kDa) was identified in EVs, and Bsflagellin conjugated Fc proteins were identified in #1 (Fc-bs flagellin, i.e., Fc-linker-bs flagellin fusion protein) and #2 (Fc-bsflagellin3, i.e., Fc-linker 3 repeat-bs flagellin fusion protein), respectively, at a size of 55 kDa. No signal was detected in TdTmt lane used for Negative control. The Fc-bsflagellin fusion protein is a fusion protein of the bsflagellin-linker (GGGGS (SEQ ID NO: 8))-Fc structure, and the Fc-bsflagellin3 refers to a fusion proteins of a bsflagellin-linker(GGGGS 3 repeats (SEQ ID No: 9))-Fc structure.

### Example 6: Confirmation of dimerization of Fc-mIgG1-bsflagellin fusion protein

An attempt was made to determine whether the Fc-mIgG1-bsflagellin fusion protein, whose expression was confirmed, forms a dimer.

As shown in FIG. 8, under Non-reducing conditions, a dimer formation signal corresponding to about twice the size from which the signal emerges at reducing conditions was observed. This confirmed that the Fc-bsflagellin fusion protein forms a dimer.

### Example 7: Comparison of the activities of Fc-flagellin and wild-type flagellin as TLR5 agonists

To confirm that the Fc-bsflagellin fusion protein prepared above exhibits activity as a TLR5 agonist, the intracellular NF-κB activity was evaluated in the same manner as in Example 4.

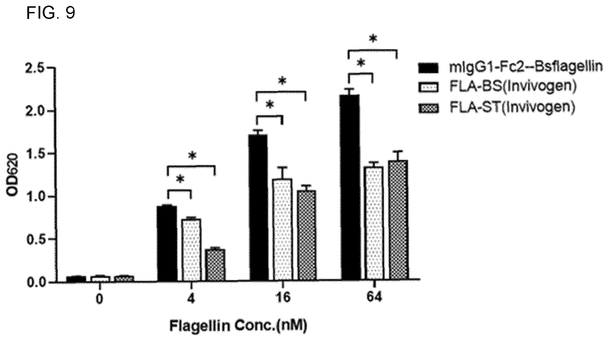

The results are shown in FIG. 9.

The activity of Toll-like receptor (TLR) 5-dependent NF-κB was confirmed with cell culture obtained 48 hours after transfection of Fc-mIgG1-Bsflagellin into HEK293T cells. For comparison, purified flagellin (FLA-BS) from *Bacillus subtilis* and purified flagellin (FLA-ST) from *Salmonella typhimurium,* sold at 99% purity by Invivogen, were used. When each test substance was treated, it was confirmed that the TLR5-dependent NF-κB activity by Fc-bsflagellin was significantly higher than that of FLA-BS or FLAs-ST used as a standard protein.

The above results indicate that flagellin fused to the Fc region of an immunoglobulin exhibits significantly improved TLR5 pathway activation ability as compared to wild-type flagellins, fragments of wild type flagelline or variants of wild-type flagellin.

The amino acid sequence of each protein used in the above experiment and the sequence of the polynucleotide encoding it are as follows:
Bsflagellin amino acid sequence: SEQ ID NO: 1
Sdflagellin amino acid sequence: SEQ ID NO: 2
Paflagellin amino acid sequence: SEQ ID NO: 3
Shflagellin amino acid sequence: SEQ ID NO: 4
Ecflagellin amino acid sequence: SEQ ID NO: 5
Human IgG4 Fc amino acid sequence: SEQ ID NO: 6
Mouse IgG1 Fc amino acid sequence: SEQ ID NO: 7
Linker 1 amino acid sequence: SEQ ID NO:8
Linker 2 amino acid sequence: SEQ ID NO: 9
hIgG4-Fc-Bsflagellin fusion protein amino acid sequence: SEQ ID NO: 10
mIgG1-Fc-Bsflagellin fusion protein (Linker 1) amino acid sequence: SEQ ID NO: 11
mIgG1-Fc-Bsflagellin fusion protein (Linker 2) amino acid sequence: SEQ ID NO: 12
hIgG4-Fc-Sdflagellin fusion protein amino acid sequence: SEQ ID NO: 13
hIgG4-Fc-Paflagellin fusion protein amino acid sequence: SEQ ID NO: 14
hIgG4-Fc-Shflagellin fusion protein amino acid sequence: SEQ ID NO: 15
hIgG4-Fc-Ecflagellin fusion protein amino acid sequence: SEQ ID NO: 16
hIgG4-Fc-Bsflagellin polynucleotide sequence: SEQ ID NO: 17
mIgG1-Fc-Bsflagellin (Linker 1) polynucleotide sequence: SEQ ID NO: 18
mIgG1-Fc-Bsflagellin (Linker 2) polynucleotide sequence: SEQ ID NO: 19
hIgG4-Fc-Sdflagellin polynucleotide sequence: SEQ ID NO: 20
hIgG4-Fc-Paflagellin polynucleotide sequence: SEQ ID NO: 21
hIgG4-Fc-Shflagellin polynucleotide sequence: SEQ ID NO:22
hIgG4-Fc-Ecflagellin polynucleotide sequence: SEQ ID NO:23

### INDUSTRIAL APPLICABILITY

The fusion protein provided by the present invention has significantly superior toll-like receptor 5 (TLR5) pathway activation ability compared to wild-type flagellin, a fragment thereof or a mutant thereof, and it can be very usefully utilized in the development of therapeutic agents for diseases that can be prevented, improved, or treated through activation of the TLR5 pathway and/or vaccine adjuvant development, and thus has very high industrial applicability.

## Claims

1. A fusion protein comprising a flagellin, a fragment thereof, or a variant thereof; and an immunoglobulin Fc region.

2. The fusion protein according to claim 1, wherein the flagellin is flagellin derived from a microorganism selected from the group consisting of the genus *Bacillus, Salmonella, Helicobacter, Vibrio, Serratia, Shigella, Treponema, Legionella, Borrelia, Clostridium, Agrobacterium, Bartonella, Proteus, Pseudomonas, Escherichia, Listeria, Yersinia, Campylobacter, Roseburia, and Marinobacter.*

3. The fusion protein according to claim 1, wherein the flagellin is flagellin derived from a microorganism selected from the group consisting of *Salmonella enteritidis, Salmonella typhimurium, Salmonella Dublin, Salmonella enterica, Helicobacter pylori, Vibrio cholera, Vibrio vulnificus, Vibrio fibrisolvens, Serratia marcesens, Shigella flexneri, Treponema pallidum, Borrelia burgdorferei, Clostridium difficile, Agrobacterium tumefaciens, Bartonella clarridgeiae, Proteus mirabilis, Bacillus subtilis, Bacillus cereus, Bacillus halodurans, Pseudomonas aeruginosa, Escherichia coli, Listeria monocytogenes, Yersinia pestis, Campylobacter spp, Roseburia spp and Marinobacter spp.*

4. The fusion protein according to claim 1, wherein the flagellin comprises a conserved sequence recognized by toll-like receptor 5 (TLR5).

5. The fusion protein according to claim 1, wherein the fragment has a hypervariable region removed from wild-type flagellin.

6. The method of claim 1, wherein the fragment comprises at least one selected from the group consisting of C-terminal domain 0, C-terminal domain 1, C-terminal domain 2, N-terminal domain 2, N-terminal domain 1, N-terminal domain 0 of wild type flagellin and a domain exhibiting at least 80% amino acid sequence homology with each of the domains.

7. The fusion protein according to claim 1, wherein the variant shows at least 80% amino acid sequence homology with wild-type flagellin and exhibits Toll-like receptor 5 (TLR5) stimulating activity.

8. The fusion protein according to claim 1, wherein the immunoglobulin Fc region is derived from an Fc of human or animal immunoglobulin IgG, IgM, IgD, IgA or IgE.

9. The fusion protein according to claim 1, wherein the immunoglobulin Fc region is derived from an Fc of human or animal immunoglobulin IgG1, IgG2, IgG3 or IgG4.

10. The fusion protein according to claim 1, wherein the immunoglobulin Fc region comprises at least one selected from the group consisting of CH1, CH2, CH3 and CH4 domains.

11. The fusion protein according to claim 10, wherein the immunoglobulin Fc region further comprises a hinge region.

12. The fusion protein according to claim 1, wherein the N-terminus or C-terminus of the flagellin, the fragment thereof or the variant thereof is bound to the N-terminus or C-terminus of the immunoglobulin Fc region.

13. The fusion protein according to claim 1, wherein the flagellin, the fragment thereof, or the variant thereof; and the immunoglobulin Fc region is linked via a linker.

14. The fusion protein according to claim 1, wherein the flagellin, the fragment thereof, or the variant thereof consists of an amino acid sequence selected from the group consisting of SEQ ID NOs: 1 to 5 or an amino acid sequence exhibiting at least 80% sequence homology thereto.

15. The fusion protein according to claim 1, wherein the immunoglobulin Fc region consists of the amino acid sequence of SEQ ID NO: 6 or 7 or an amino acid sequence exhibiting at least 80% sequence homology thereto.

16. The fusion protein according to claim 13, wherein the linker consists of the amino acid sequence of SEQ ID NO: 8 or SEQ ID NO: 9.

17. The fusion protein according to claim 1, wherein the fusion protein consists of an amino acid sequence selected from the group consisting of SEQ ID NOs: 10 to 16.

18. A polynucleotide encoding the fusion protein of any one of claims 1 to 17.

19. A vector comprising the polynucleotide of claim 18 .

20. A transformant transformed with the vector of claim 19 .

21. A pharmaceutical composition comprising the fusion protein of any one of claims 1 to 17 as an active ingredient.

22. The pharmaceutical composition according to claim 21, wherein the pharmaceutical composition exhibits Toll-like receptor 5 (TLR5) stimulating activity.

23. The pharmaceutical composition according to claim 21, wherein the pharmaceutical composition is for the prevention or treatment of damage by exposure to radiation; for the prevention or treatment of reperfusion injury; for prevention or treatment of inflammatory bowel disease; for prevention or treatment of autoimmune diseases; for prevention or treatment of viral infection; for prevention or treatment of metabolic diseases; for prevention or treatment of aging; or for enhancement of immune function, or for prevention or treatment of cancer.

24. The pharmaceutical composition according to claim 23, wherein the damage caused by radiation exposure is gastrointestinal syndrome or hematopoietic syndrome.

25. The pharmaceutical composition according to claim 23, wherein the aging is at least one selected from the group consisting of hair loss due to aging, cataracts, hernias, colitis, osteoporosis, and osteomalacia.

26. A vaccine adjuvant comprising the fusion protein of any one of claims 1 to 17 as an active ingredient.

27. Use of the fusion protein of any one of claims 1 to 17 for the preparation of a pharmaceutical agent for the treatment of injury by radiation exposure; the treatment of reperfusion injury; the treatment of inflammatory bowel disease; the treatment of autoimmune disease; the treatment of viral infection; the treatment of metabolic disease; the treatment of aging; the enhancement of immune function; or the treatment of cancer.

28. A method for treating injury by exposure to radiation; treating reperfusion injury; treating inflammatory bowel disease; treating autoimmune disease, treating viral infection; treating metabolic disease; treating aging; enhancing immune function; or treating cancer, comprising administering to a subject in need thereof an effective amount of a composition comprising the fusion protein of any one of claims 1 to17 as an active ingredient.
